(19) **Europäisches Patentamt**
European Patent Office
Office européen des brevets

(11) **EP 2 729 099 B1**

(12) **EUROPÄISCHE PATENTSCHRIFT**

(45) Veröffentlichungstag und Bekanntmachung des Hinweises auf die Patenterteilung:
**20.11.2019 Patentblatt 2019/47**

(21) Anmeldenummer: **11743185.8**

(22) Anmeldetag: **04.07.2011**

(51) Int Cl.:
*A61F 9/008* (2006.01)      *A61F 9/009* (2006.01)
*A61B 34/20* (2016.01)

(86) Internationale Anmeldenummer:
**PCT/EP2011/003313**

(87) Internationale Veröffentlichungsnummer:
**WO 2013/004255 (10.01.2013 Gazette 2013/02)**

(54) **VORRICHTUNG UND VERFAHREN FÜR EIN LASERGESTÜTZTES AUGENCHIRURGISCHES BEHANDLUNGSSYSTEM**

DEVICE AND METHOD FOR A LASER-ASSISTED EYE SURGERY TREATMENT SYSTEM

DISPOSITIF ET PROCÉDÉ POUR UN SYSTÈME DE TRAITEMENT CHIRURGICAL OCULAIRE ASSISTÉ PAR UN LASER

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**14.05.2014 Patentblatt 2014/20**

(73) Patentinhaber: **WaveLight GmbH**
**91058 Erlangen (DE)**

(72) Erfinder:
• **DONITZKY, Christof**
**90542 Eckental/Eschenau (DE)**
• **WUELLNER, Christian**
**91096 Moehrendorf (DE)**

• **RIEDEL, Peter**
**90489 Nuernberg (DE)**

(74) Vertreter: **Katérle, Axel**
**Wuesthoff & Wuesthoff**
**Patentanwälte PartG mbB**
**Schweigerstraße 2**
**81541 München (DE)**

(56) Entgegenhaltungen:
**EP-A2- 1 767 174          WO-A1-2006/060323**
**WO-A1-2011/035063     WO-A2-03/049656**
**US-A1- 2003 223 037    US-A1- 2008 051 772**
**US-A1- 2010 256 614    US-B2- 6 866 661**

EP 2 729 099 B1

**Beschreibung**

Die Erfindung betrifft eine Vorrichtung sowie ein Verfahren zum Einsatz in einem lasergestützten augenchirurgischen Behandlungssystem.

In der refraktiven ophthalmologischen Chirurgie werden durch Eingriffe am Auge eines Patienten die Brechungs- und damit die Abbildungseigenschaften des Auges verändert, um Sehfehler zu lindern oder zu korrigieren. Eine bekannte Operationsform ist beispielsweise die LASIK (Laser in-situ Keratomileusis). Hierbei wird ein flacher Hornhautschnitt erzeugt, durch den ein als Deckel dienendes Scheibchen (in der Fachwelt üblicherweise mit dem englischen Begriff Flap bezeichnet) erzeugt wird, das an einer Stelle fest mit der Hornhaut verbunden bleibt und dort ein Scharnier (üblicherweise als Hinge bezeichnet) bildet. An diesem Scharnier kann der Flap aufgeklappt werden. Dadurch kann das unter dem Flap liegende Hornhautgewebe freigelegt werden, in dem dann mittels geeigneter Laserstrahlung - üblicherweise Strahlung eines Excimerlasers - eine Gewebeablation nach Maßgabe eines abhängig vom Sehfehler bestimmten Abtragsprofils vorgenommen werden kann. Anschließend wird der Flap zurückgeklappt. Da das Epithel weitgehend unverletzt bleibt, erfolgt der Heilungsprozess relativ kurz und schmerzfrei.

Bei der klassischen LASIK wird der Flap mittels eines mechanischen Mikrokeratoms geschnitten. Es ist allerdings auch bekannt, den Flap mittels geeigneter Laserstrahlung zu schneiden. Diese Variante ist in der Fachwelt üblicherweise unter dem Begriff Femto-LASIK oder Fs-LASIK bekannt, weil dort bisher üblicherweise gepulste Laserstrahlung mit Pulsdauern im Bereich von Femtosekunden eingesetzt wird. Es ist freilich darauf hinzuweisen, dass Gewebeschnitte in der humanen Kornea auch mit kürzeren oder längeren Pulsdauern angebracht werden können, etwa im Atto- oder Pikosekundenbereich. Deshalb wird nachfolgend der Begriff lasergestützte LASIK verwendet, wenn es um eine LASIK-Form geht, bei welcher der Flapschnitt lasertechnisch erzeugt wird.

Die hier offenbarte Erfindung ist grundsätzlich bei verschiedenen Behandlungsformen einsetzbar, bei denen Laserstrahlung in Bezug auf das Auge in definierter Weise zu positionieren ist. Darunter fallen nicht nur die lasergestützte LASIK, sondern beispielsweise auch die lasergestützte Keratoplastik (lamellär oder perforierend), die lasergestützte korneale Lentikelextraktion und weitere Operationsformen, welche die Anbringung intrakornealer Schnitte erfordern.

Lasersysteme, mit denen Schnitte im humanen Auge erzeugt werden können, weisen typischerweise einen sogenannten Applikator auf, der ein für die verwendete Laserstrahlung transparentes Kontaktelement umfasst, welches eine Kontaktfläche zur flächigen Anlage des zu behandelnden Auges bereitstellt. Solche Applikatoren werden oftmals auch als Patienteninterface bezeichnet, weil sie eine (mechanische) Schnittstelle des Lasersystems zum Auge bieten. Der Applikator kann seinerseits lösbar beispielsweise mit einem Fokussierobjektiv des Lasersystems gekoppelt sein. Indem der Applikator so in Eingriff mit dem Auge gebracht wird, dass sich die Augenoberfläche an die Kontaktfläche anschmiegt, kann das Auge gegenüber dem Koordinatensystem der für die räumliche Steuerung des Strahlungsfokus verantwortlichen Komponente des Lasersystems referenziert werden. Auf diese Weise ist eine präzise Schnitterzeugung an der gewünschten Stelle im Auge möglich.

Vor der Durchführung einer der Erzeugung eines Schnitts (oder allgemeiner ausgedrückt einer Schnittfigur) im Auge dienenden Laserbehandlung stellt sich das generelle Problem, dass der Patient mit seinem zu behandelnden Auge gegenüber dem Lasersystem ausgerichtet werden muss. Die Schnittfigur soll in der Regel an einer bestimmten Position im Auge und auch - soweit es sich nicht um eine rotationssymmetrische Figur handelt - in einer bestimmten Orientierung zum Auge erzeugt werden. Für einen Flapschnitt im Rahmen einer LASIK-Behandlung beispielsweise gilt die Maßgabe, dass er dort im Auge erzeugt werden soll, wo später Gewebe ablatiert werden soll, d.h. der Flap muss das zu ablatierende Gebiet überdecken. Zugleich besitzt ein Flapschnitt eine Unsymmetrie in Form des Scharniers. Vor allem wenn das zu ablatierende Gebiet ebenfalls rotationsunsymmetrisch ist, beispielsweise weil ein Astigmatismus behoben werden soll, ist es leicht nachvollziehbar, dass das Flapscharnier in einer bestimmten optimalen Lage gegenüber dem vorgesehenen Ablationsgebiet liegen sollte, um bei kleinstmöglicher Flapgröße dennoch ungehindert die Ablation in allen vorgesehenen Bereichen durchführen zu können. Im Fall des Astigmatismus beispielsweise kann es erwünscht und notwendig sein, das Flapscharnier in bestimmter Weise gegenüber dem astigmatischen Teil der Hornhautoberfläche, d.h. gegenüber der Achse des Astigmatismus, auszurichten. Die erforderliche Ausrichtung des Patientenauges gegenüber dem Lasersystem kann sich daher nicht nur auf die Position, sondern auch auf die Orientierung des Auges relativ zu dem Lasersystem beziehen. Orientierung meint hier ganz generell die Ausrichtung einer ersten Achse, welche eine Ausdehnungsrichtung eines ersten ausgedehnten Objekts kennzeichnet, gegenüber einer zweiten Achse, welche eine Ausdehnungsrichtung eines zweiten ausgedehnten Objekts kennzeichnet, oder gegenüber einem gegebenen Koordinatensystem.

Bisher ist es in der Regel üblich, dass diese Ausrichtung des Patienten gegenüber dem Lasersystem händisch und nach Augenmaß, ggf. unter Zuhilfenahme eines Mikroskops, vom behandelnden Arzt durchgeführt wird. Er versucht dabei üblicherweise, den Applikator möglichst zentrisch in Bezug auf bestimmte Konturen des Auges anzuordnen und ihn in seiner Orientierung in Bezug auf bestimmte Konturen des Auges auszurichten. Dies kann beispielsweise durch entsprechende Ausrichtung einer Patientenliege, auf welcher der Patient liegt, oder/und durch entsprechende Manövrierung eines den Applikator tragenden Strahlarms des Lasersystems geschehen. Weil der Applikator seinerseits nur in einer bestimmten Orientierung an dem Lasersystem anbringbar ist, bedeutet diese Orientierungsausrichtung des Applikators gegenüber dem Auge zugleich eine Orientierungsausrichtung des Lasersystems als Ganzes gegenüber dem Auge.

Nachteilig an dieser Vorgehensweise ist, dass die Qualität der Ausrichtung von der Erfahrung und den Fähigkeiten des Arztes abhängt und damit mehr oder weniger starken Schwankungen unterliegen kann. Außerdem erfordert die manuelle Ausrichtung durch den Arzt eine vergleichsweise lange Zeit. Ziel ist es jedoch generell, die gesamte Operationszeit möglichst kurz zu halten, um die Unannehmlichkeiten für den Patienten möglichst gering zu halten. Je länger der Vorgang des Andockens des Auges an den Applikator dauert, weil in dieser Phase der Arzt mühsam die richtige Ausrichtung des Applikators gegenüber dem Auge einstellen und überwachen muss, desto länger dauert die Operation insgesamt.

Zum relevanten Stand der Technik wird verwiesen auf die Dokumente US 2003/223037 A1, EP 1 767 174 A2, WO 2011/035063 A1 und US 6,866,661 B2.

Eine Aufgabe von Ausführungsformen der Erfindung ist es, bei einem für die lasergestützte Anbringung intraokularer Schnitte geeigneten Lasersystem den Andockvorgang eines Applikators des Lasersystems an das Auge zu verkürzen. Erfindungsgemäß sind eine Vorrichtung und ein Verfahren mit den Merkmalen der unabhängigen Ansprüche 1 und 6 vorgesehen. Bevorzugte Ausgestaltungen sind in den abhängigen Ansprüchen definiert.

Die Vorrichtung ist zum Einsatz in einem lasergestützten augenchirurgischen Behandlungssystem vorgesehen und umfasst eine erste Bilderfassungseinheit, die dazu eingerichtet ist, ein erstes Bild eines zu behandelnden Auges zu erfassen. Ferner umfasst die Vorrichtung eine Rechneranordnung, die dazu eingerichtet ist, die Durchführung der folgenden Schritte zu bewirken:

(i) durch Bildverarbeitung des ersten Bilds mindestens ein erstes Merkmal des Auges zu erkennen und eine Position und eine Orientierung des ersten Merkmals in einem Koordinatensystem des Behandlungssystems zu ermitteln,
(ii) eine Position und eine Orientierung einer in dem Auge zu erzeugenden Schnittfigur in dem Koordinatensystem des Behandlungssystems abhängig von der ermittelten Position und Orientierung des ersten Merkmals in dem Koordinatensystem sowie abhängig von einer vorab ermittelten relativen Position und Orientierung mindestens eines zweiten Merkmals des Auges gegenüber dem ersten Merkmal zu ermitteln.

Das erste Merkmal kann sich beispielsweise auf eine durch Bilderfassung detektierbare Augenstruktur wie etwa die Iris, die Pupille, das Pupillenzentrum, den Limbus, eine sklerale Blutgefäßanordnung und/oder eine korneale Dickenverteilung beziehen. Das zweite Merkmal kann sich beispielsweise auf einen astigmatisch verformten Hornhautbereich beziehen, der durch eine Astigmatismusachse repräsentierbar ist. Das zweite Merkmal kann gewünschtenfalls von Eigenschaften des ersten Merkmals abhängen. Beispielsweise ist es vorstellbar, aus einer kornealen Dickenverteilung einen astigmatisch verformten Hornhautbereich zu ermitteln. Dies sind selbstverständlich nur Beispiele, die keinesfalls beschränkend zu verstehen sind. Es sind auch andere detektierbare Augenmerkmale als erstes bzw. zweites Merkmal vorstellbar. Insbesondere sind mittels optischer Kohärenztomographie (optical coherence tomography, OCT) erfasste Schichtmerkmale des Auges denkbar.

Im Fall einer LASIK-Operation kann präoperativ an einer Diagnosestation beispielsweise ein Irisbild des zu behandelnden Auges erfasst werden und aus dem Irisbild eine geeignete Augenstruktur (z.B. ein bestimmtes sklerales Blutgefäß oder eine korneale Dickenverteilung) als erstes Merkmal erkannt werden. Zugleich können an der Diagnosestation mit einem Keratometer die Topographie der Korneavorderfläche untersucht werden und Keratometerwerte ermittelt werden, welche die Achslage und Stärke eines kornealen Astigmatismus repräsentieren. Zusätzlich oder alternativ kann an der Diagnosestation mittels optischer Kohärenztomographie (optical coherence tomography, OCT) oder mittels einer Scheimpflugmessung eine pachymetrische Aufnahme des Auges gemacht werden, aus der die korneale Dickenverteilung ermittelt wird. Das astigmatische Gebiet der Kornea kann als zweites Merkmal dienen. Soweit die Irisaufnahme, die Keratometrie und die Pachymetrie an der gleichen Diagnosestation vorgenommen werden, ist davon auszugehen, dass das Irisbild in einem bekannten Bezug zu der bei der Keratometrie und/oder Pachymetrie ermittelten Achslage des Astigmatismus liegt. Es können daher Referenzinformationen ermittelt werden, welche die relative Position und die relative Orientierung des Astigmatismusgebiets gegenüber dem ersten Merkmal repräsentieren, beispielsweise in vektorieller Form.

Für die eigentliche LASIK-Operation kann der Patient von der Diagnosestation an eine Behandlungsstation verlegt werden, die sich beispielsweise in einem anderen Raum der ärztlichen Praxis befindet. Die LASIK-Operation soll in diesem Beispielfall das Ziel haben, eine durch den oben gemessenen Astigmatismus verursachte Sehschwäche des Auges zu korrigieren. Das bedeutet, dass der Flap so geschnitten werden muss, dass er das Astigmatismusgebiet überdeckt, so dass nach Zurückklappen des Flaps das Astigmatismusgebiet mittels Laserstrahlung ablatierend behandelt werden kann. Hierbei kommt es insbesondere auf eine geeignete relative Lage des Flapscharniers zum Astigmatismusgebiet an. Es sollte insbesondere sichergestellt sein, dass das Flapscharnier außerhalb desjenigen Bereichs liegt, der ablatiert werden müsste, um den Sehfehler zu korrigieren. Das heißt, der Flap muss hinsichtlich seiner Position und Orientierung (ggf. auch hinsichtlich seiner Größe) an die Lage des Astigmatismusgebiets angepasst werden.

Im Fall eines Flaps kann es wünschenswert sein, dass die Schnittfigur einen mit einer Schnittfläche des Flaps in Verbindung stehenden Hilfskanal definiert, der durch einen entsprechenden Hilfsschnitt als Teil der Schnittfigur gebildet wird. Ein solcher Hilfskanal, der vorzugsweise im Bereich des Flapscharniers mit besagter Schnittfläche des Flaps in Verbindung steht, kann zur Abführung von Gasen dienen, welche bei der photodisruptiven Schnitterzeugung im Augengewebe entstehen. Der Hilfskanal erstreckt sich weg von dem Flap und kann beispielsweise bis mindestens in den Bereich des Limbus des behandelten Auges reichen. An seinem Flap-fernen Ende kann er an der Augenoberfläche austreten oder er kann in der Tiefe des Augengewebes enden. Beispielsweise kann er bis unter die Bindehaut des Auges reichen oder sich die Sklera des Auges hinein erstrecken.

Um sicherzustellen, dass der Hilfskanal stets die Forderung erfüllt, bis mindestens in den Bereich des Limbus sich zu erstrecken, ist in einer vorteilhaften Weiterbildung der Erfindung vorgesehen, dass die Rechneranordnung dazu eingerichtet ist, abhängig von der ermittelten Position und Orientierung zumindest solcher Teile der Schnittfigur, die den Flap definieren, Steuerdaten für die Erzeugung des Hilfskanals derart zu erzeugen, dass sich der Hilfskanal bis mindestens in den Bereich des Limbus des Auges und vorzugsweise sogar bis jenseits des Limbus erstreckt. Dies ermöglicht es, die nötigen Steuerdaten für die Steuerung der Laserstrahlung stets in Anpassung an die ermittelte Position und Orientierung des Flaps, genauer die ermittelte Position und Orientierung derjenigen Elemente der Schnittfigur, die den Flap definieren, zu erzeugen. Dies kann insbesondere eine geeignete Anpassung der Länge des Hilfskanals erfordern, so dass sich dieser sicher bis zum Limbus oder darüber hinaus erstreckt.

Der Hilfskanal kann beispielsweise von einem im wesentlichen ebenen Schnitt gebildet werden. Er kann über seine Länge im wesentlichen gleichbleibende Breite besitzen, kann aber hinsichtlich seiner Breite auch variieren, beispielsweise vom Flap aus in Richtung zu seinem anderen Ende zunehmend breiter oder alternativ zunehmend schmaler werden.

Zweckmäßigerweise wird der Hilfskanal erzeugt, bevor die den Flap definierenden Elemente der Schnittfigur geschnitten werden.

Alternativ zu einem LASIK-Flap kann die Schnittfigur ein zu extrahierendes korneales Lentikel definieren. Durch Extraktion eines geeignet geformten Gewebestücks aus dem Inneren der Kornea kann ebenfalls eine refraktive Korrektur von Fehlsichtigkeiten des Auges erzielt werden. Weil dieses Gewebestück typischerweise annähernd linsenförmig ist, wird es auch als Lentikel bezeichnet. Da sich die Geometrie des Lentikels nach der zu behebenden Fehlsichtigkeit des Auges richtet und diese oftmals nicht exakt rotationssymmetrisch ist, sondern beispielsweise einen Astigmatismus beinhaltet, eignet sich auch die korneale Lentikelextraktion zur Anwendung der Erfindung, indem die das Lentikel definierende Schnittfigur hinsichtlich Position und Orientierung, ggf. auch hinsichtlich Form oder/und Größe, an einem geeigneten zweiten Merkmal, etwa einem astigmatisch verformten Hornhautbereich, ausgerichtet wird. Alternativ oder zusätzlich kann die Position, Orientierung und/oder Größe der Lentikel-Schnittfigur an der - bildtechnisch erfassten - Lage des Pupillenzentrums des Auges und/oder der kornealen Dickenverteilung des Auges ausgerichtet werden.

An der Behandlungsstation kann das zu behandelnde Auge des Patienten in Kontakt mit einem Applikator des Lasersystems gebracht werden, so dass das Auge gegenüber dem Applikator fixiert ist. Mittels einer Kamera des Lasersystems kann sodann ein Bild des Auges erfasst werden, wobei ein Rechner des Lasersystems mittels geeigneter Bildverarbeitungssoftware dieses Bild auswerten und darin das erste Merkmal, etwa ein bestimmtes sklerales Blutgefäß oder eine korneale Dickenverteilung, erkennen kann. Sobald das erste Merkmal erkannt ist, kann der Rechner die Position und Orientierung dieses Merkmals in einem Koordinatensystem des Lasersystems ermitteln. Anhand der erwähnten Referenzinformationen kann der Rechner sodann die Position und Orientierung des zweiten Merkmals in dem Koordinatensystem des Lasersystems ermitteln. Basierend auf der so erworbenen Kenntnis über die Position und Orientierung des zweiten Merkmals (Astigmatismusgebiet) in dem Koordinatensystem des Lasersystems kann der Rechner daraufhin eine geeignete Schnittfigur für den Flap und ggf. den Hilfskanal bestimmen. Insbesondere kann er die Position und Orientierung des Flapscharniers in dem Koordinatensystem des Lasersystems geeignet festlegen und er kann auch eine geeignete Form oder/und Größe des Flaps bestimmen.

Weil bei dieser Vorgehensweise kein händisches Ausrichten des Patientenauges gegenüber dem Lasersystem durch den Operateur erforderlich ist, sondern stattdessen die Position und Orientierung der Schnittfigur rechnergestützt in automatischer Weise angepasst werden können, kann die Phase vom Andocken des Applikators an das Auge bis zum tatsächlichen Beginn des Schneidvorgangs der Schnittfigur kurz gehalten werden. Dies verringert die für den Patienten mit der Operation verbundenen Unannehmlichkeiten.

Wie bereits erwähnt, kann die Erfindung beispielsweise bei LASIK-Operationen Anwendung finden. Hierfür kann die Schnittfigur einen kornealen Flap mit einem Flapscharnier und ggf. einem als Gasentlüftungskanal dienenden Hilfskanal definieren.

In einer bevorzugten Ausgestaltung der Erfindung kann die Rechneranordnung dazu eingerichtet sein, die Position und Orientierung eines das Flapscharnier definierenden Figurelements der Schnittfigur abhängig von der Position und Orientierung des ersten Merkmals in dem Koordinatensystem des Behandlungssystems sowie abhängig von der relativen Position und Orientierung des zweiten Merkmals gegenüber dem ersten Merkmal zu ermitteln.

Soweit das mindestens eine zweite Merkmal einen astigmatisch gekrümmten Hornhautbereich umfasst, kann die Rechneranordnung dazu eingerichtet sein, die Position und Orientierung der Schnittfigur unter Berücksichtigung einer vorgegebenen Soll-Lagebedingung zwischen dem Flap und dem astigmatisch gekrümmten Hornhautbereich zu ermitteln.

Beispielsweise kann dem Flapscharnier eine Scharnierachse zugeordnet sein und es kann dem astigmatisch gekrümmten Hornhautbereich eine Astigmatismusachse zugeordnet sein. Die Rechneranordnung kann dann dazu eingerichtet sein, die Position und Orientierung der Schnittfigur unter Berücksichtigung einer vorgegebenen Soll-Lagebedingung zwischen der Scharnierachse und der Astigmatismusachse zu ermitteln. Diese Soll-Lagebedingung zwischen der Scharnierachse und der Astigmatismusachse kann beispielsweise eine zueinander im wesentlichen senkrechte Lage der beiden Achsen vorgeben.

Die erfindungsgemäße Vorrichtung kann ein Diagnosegerät mit einer zweiten Bilderfassungseinheit zur Erfassung eines zweiten Bilds des zu behandelnden Auges umfassen. Dieses Diagnosegerät kann dazu eingerichtet sein, in dem zweiten Bild durch Bildverarbeitung das mindestens eine Merkmal zu erkennen und Merkmalsinformationen betreffend eine Position und Orientierung jedes der beiden Merkmale zu erzeugen. Daten hinsichtlich Position und Orientierung des zweiten Merkmals kann das Diagnosegerät beispielsweise anhand von topographischen Messungen der Korneavorderfläche und/oder -rückfläche mittels eines Keratometers oder anhand einer Messung der kornealen Dickenverteilung mittels optischer Kohärenztomographie (OCT) oder einer mittels einer Scheimpflug-Kamera gewinnen. Die Rechneranordnung oder schon das Diagnosegerät selbst kann dazu eingerichtet sein, auf Grundlage der Merkmalsinformationen die relative Position und Orientierung des zweiten Merkmals gegenüber dem ersten Merkmal zu ermitteln.

Es ist vorstellbar, dass das Diagnosegerät und die erste Bilderfassungseinheit verschiedenen Arbeitsstationen in einer ärztlichen Praxis zugeordnet sind.

Dem Diagnosegerät kann eine Datenbank zugeordnet sein, um darin die Merkmalsinformationen oder/und hiervon abgeleitete Informationen in Zuordnung zu Patientenkenninformationen abzulegen. Die Rechneranordnung kann dabei Zugang zu der Datenbank besitzen, so dass sie anhand der Merkmalsinformationen die relative Position und Orientierung des zweiten Merkmals gegenüber dem ersten Merkmal ermitteln kann. Es ist gleichermaßen vorstellbar, dass das Diagnosegerät selbst dazu eingerichtet ist, die relative Position und Orientierung des zweiten Merkmals gegenüber dem ersten Merkmal anhand der Merkmalsinformationen zu ermitteln. In diesem Fall kann eine Ausgestaltung vorgesehen sein, bei welcher das Diagnosegerät entsprechende Informationen über die relative Position und Orientierung des zweiten Merkmals gegenüber dem ersten Merkmal in einer Datenbank ablegt, auf welche die Rechneranordnung zugreifen kann.

Gemäß einer bevorzugten Weiterbildung kann die Rechneranordnung dazu eingerichtet sein, eine bildliche Darstellung der Schnittfigur zu bewirken, welche die ermittelte Position und Orientierung der Schnittfigur in Relation zu dem ersten Merkmal oder/und dem zweiten Merkmal oder/und einem zu ablatierenden Korneabereich veranschaulicht. Die bildliche Darstellung ermöglicht es einem Operateur, vor Operationsbeginn anhand der eigenen Anschauung zu überprüfen, ob die von der Rechneranordnung vorgeschlagene Position und Orientierung der Schnittfigur im konkreten Fall geeignet ist. Beispielsweise kann die Rechneranordnung dazu eingerichtet sein, die bildliche Darstellung auf einem Monitor oder/und durch Einblendung in einen Beobachtungsstrahlengang eines Operationsmikroskops zu bewirken.

Es kann nicht ausgeschlossen werden, dass der Operateur mit dem Vorschlag für die Position und Orientierung der Schnittfigur nicht einverstanden ist und Modifikationen vornehmen möchte. Zu diesem Zweck kann die Rechneranordnung dazu eingerichtet sein, die ermittelte Position oder/und Orientierung der Schnittfigur nach Maßgabe einer Benutzereingabe zu modifizieren und die bildliche Darstellung der Schnittfigur gemäß der modifizierten Position oder/und Orientierung zu modifizieren.

Es ist vorteilhaft, wenn die Rechneranordnung dazu eingerichtet ist, eine benutzerseitig eingegebene Bestätigung für die Position und die Orientierung der Schnittfigur zu empfangen und abhängig vom Empfang dieser Bestätigung Steuerdaten für eine Lasereinrichtung zu erzeugen und die Lasereinrichtung nach Maßgabe dieser Steuerdaten zur Erzeugung der Schnittfigur in dem Auge zu steuern.

Das erste Merkmal oder/und das zweite Merkmal können, jedenfalls was die Ermittlung ihrer Position und Orientierung anbelangt, beispielsweise jeweils durch mehrere (beispielsweise zwei oder drei) Punkte repräsentiert werden, die man auch als Pixel oder Vektor bezeichnen kann. Beide Merkmale sind vorzugsweise nicht-punktförmige, wiedererkennbare Objekte/Strukturen, die eine Strecke oder eine Fläche in einem kameratechnisch, topographisch oder anderweitig erzeugten Bild aufspannen und beispielsweise eine Vorzugsausdehnung besitzen. Jedes Pixel eines solchen Bilds kann beispielsweise durch zwei oder drei Koordinatenwerte und/oder durch mindestens einen Grauwert oder einen Farbwert definiert sein.

Die Merkmalsinformationen können beispielsweise für das erste und das zweite Merkmal jeweils einen Datensatz umfassen, der eine Mehrzahl (beispielsweise drei) charakteristischer Punkte des ersten bzw. des zweiten Merkmals in einem beliebigen Koordinatensystem beschreibt. Die Punkte des ersten Merkmals können dabei durch drei Vektoren dargestellt sein, wobei zwei relative Vektoren zwischen einem ersten und einem zweiten dieser charakteristischen Punkte sowie zwischen dem ersten und einem dritten der charakteristischen Punkte zwei linear unabhängige Koordinatenvektoren darstellen können, die ein augeninternes Koordinatensystem des Auges aufspannen und als dessen Repräsentanten dienen können. Jeder charakteristische Punkt des zweiten Merkmals kann durch die Position des ersten charakteristischen Punkts des ersten Merkmals und durch eine Linearkombination der beiden erwähnten Koordinatenvektoren eindeutig innerhalb des augeninternen Koordinatensystems festgelegt werden. Dies ermöglicht es, die Positionen der charakteristischen Punkte des zweiten Merkmals auf das durch das erste Merkmal definierte, augeninterne Koordinatensystem des Auges zu referenzieren. Somit ist es möglich, eine relative Position sowie eine relative Orientierung des zweiten Merkmals gegenüber dem ersten Merkmal eindeutig festzulegen.

An der Behandlungsstation genügt es, wenn in dem von der ersten Bilderfassungseinheit erfassten Bild lediglich das erste Merkmal erkannt und dessen Position und Orientierung in einem gegebenen Koordinatensystem des Behandlungssystems ermittelt werden. Vorab gewonnene Informationen über die relative Position und Orientierung des zweiten Merkmals gegenüber dem ersten Merkmal erlauben es dann der Rechneranordnung, die Position und Orientierung des zweiten Merkmals in dem Koordinatensystem des Behandlungssystems eindeutig zu ermitteln, ohne dabei das zweite Merkmal selbst erkennen zu müssen. Die Merkmalsinformationen erlauben es mithin, die Position und Orientierung des zweiten Merkmals in dem Koordinatensystem des Behandlungssystems rein rechnerisch zu bestimmen, ohne eine besondere Bilderfassung (etwa mit einer Kamera oder einem Topographiemessgerät) für das zweite Merkmal an der Behandlungsstation durchführen zu müssen. Dies führt zu einem Zeitgewinn bei der operativen Behandlung, da die Erkennung des zweiten Merkmals im Rahmen einer Voruntersuchung an einer Diagnosestation stattfinden kann, bei der Behandlung selbst eine solche Erkennung aber nicht erforderlich ist.

Vorteilhafterweise werden die Position und Orientierung (gewünschtenfalls auch die geometrische Form oder/und die Größe) der Schnittfigur von der Rechneranordnung abhängig von der ermittelten Position und Orientierung des zweiten Merkmals in dem Koordinatensystem des Behandlungssystems geeignet bestimmt, damit mittels der Schnittfigur direkt (z.B. bei einer intrakornealen Lentikelextraktion oder einer Keratoplastik) oder indirekt (etwa bei einer lasergestützten LASIK-Behandlung) eine Behandlung zu ermöglichen, die eine mit dem zweiten Merkmal verbundene Sehschwäche erfolgreich korrigiert.

Die Rechneranordnung kann dazu eingerichtet sein, die Position und Orientierung (gegebenenfalls auch die Form oder/und Größe) der Schnittfigur derart zu ermitteln, dass im Fall einer Flaperzeugung ein kürzester Abstand der Scharnierachse des Flapscharniers von einem Zentrum des Auges einen vorgegebenen, von Null verschiedenen Wert aufweist. Dieser kürzeste Abstand bzw. dessen vorgegebener Wert kann an die geometrische Form einer Ablationszone angepasst sein, in welcher eine korneale Ablation von Augengewebe durchgeführt werden soll. Das Zentrum des Auges kann beispielsweise durch das Pupillenzentrum oder durch eine andere Struktur des Auges definiert sein bzw. auf eine solche Struktur referenziert sein und von der Rechneranordnung aus dem von der ersten Bilderfassungseinheit erfassten ersten Bild ermittelt werden.

Das Diagnosegerät kann mit einem Kamerasystem zur Erfassung des zweiten Bilds sowie mit einer Topographiemesseinrichtung ausgestattet sein, die es erlaubt, die Topographie der Kornea zu ermitteln und anhand der so ermittelten Topographiedaten die Position und Orientierung eines bestimmten Hornhautbereichs, etwa eines astigmatisch verformten Hornhautbereichs, zu bestimmen. Die Position und Orientierung dieses Hornhautbereichs (zweites Merkmal) können dabei vorteilhafterweise auf ein augeninternes Koordinatensystem referenziert werden, welches durch das erste Merkmal des Auges definiert ist. Dieses erste Merkmal kann anhand der Bilder des Kamerasystems erkannt und positions- und orientierungsmäßig erfasst werden.

Die bildliche Darstellung der Schnittfigur gemäß ihrer ermittelten Position und Orientierung (und gegebenenfalls ihrer ermittelten Form und Größe) kann beispielsweise die Umrisskontur eines Flaps (d.h. Flapscharnier und Flaprand) veranschaulichen. Insbesondere kann es vorteilhaft sein, wenn die bildliche Darstellung nicht nur die Schnittfigur oder jedenfalls relevante Teile der Schnittfigur veranschaulicht, sondern auch das zweite Merkmal. Eine solche Veranschaulichung des zweiten Merkmals kann beispielsweise im Fall eines astigmatisch verformten Hornhautbereichs durch eine linienhafte Darstellung der Umrisskontur des astigmatischen Bereichs oder/und durch eine Darstellung einer Astigmatismusachse bewirkt werden. Dies ermöglicht dem Operateur eine besonders einfache Überprüfung, ob die von der Rechneranordnung ermittelte Schnittfigur angesichts der Lage des astigmatischen Hornhautbereichs (oder allgemein: angesichts der Lage des zweiten Merkmals) geeignet ist. Die bildliche Darstellung kann beispielsweise in ein Bild des Auges eingeblendet werden, wobei dieses Bild eines sein kann, das von einer Kamera an der Behandlungsstation aufgenommen wird, oder es kann eines sein, das der Operateur durch ein Operationsmikroskop sieht. Im ersteren Fall ist eine Visualisierung der bildlichen Darstellung auf einem Monitor zweckmäßig, im letzteren Fall kann die bildliche Darstellung durch eine geeignete Einblendeinrichtung (nach Art eines Head-Up-Displays, HUD) in den Beobachtungsstrahlengang des

**EP 2 729 099 B1**

Operationsmikroskops eingeblendet werden.

Es kann eine Eingabevorrichtung vorgesehen sein, über welche der Operateur oder ein Assistent durch manuelle Eingabe die zuvor von der Rechneranordnung ermittelte Schnittfigur modifizieren kann. Zweckmäßigerweise wird eine solche Modifikation der Schnittfigur in der bildlichen Darstellung derselben reflektiert, d.h. die bildliche Darstellung wird angepasst, sobald der Benutzer über die Eingabevorrichtung Modifikationen der Schnittfigur vornimmt. Die von der Rechneranordnung ermittelte Position und Orientierung der Schnittfigur kann mithin vom Operateur als Vorschlag aufgefasst werden, den er nach eigenem Wunsch modifizieren kann. Eine Freigabe des Lasersystems zur Erzeugung der Schnittfigur kann dabei eine Bestätigungseingabe seitens des Operateurs erfordern, gleichgültig ob der Operateur den Vorschlag der Rechneranordnung unmittelbar übernehmen möchte oder ob er zuvor noch Modifikationen anbringen möchte. Die vom Operateur vornehmbaren Modifikationen können sich nicht nur auf die Position und Orientierung der Schnittfigur beziehen, es kann darüber hinaus denkbar sein, dass der Operateur über die Eingabevorrichtung auch die geometrische Form oder/und die Größe der Schnittfigur individuell modifizieren kann.

Vorstehend wurde ein astigmatisch verformter Hornhautbereich als Beispiel für ein zweites Merkmal im Sinne der Erfindung angegeben. Es versteht sich, dass andere Strukturen oder Bereiche des Auges als zweites Merkmal dienen können, etwa ein Katarakt in der humanen Linse, falls die Erfindung im Rahmen einer Katarakt-Operation eingesetzt werden soll, bei der die Schnittfigur eine lasertechnisch zu erzeugende Inzision in der humanen Linse und/oder dem Kapselsack umfassen soll, die etwa als Zugang zur Linse dient. Insoweit ist die Erfindung keineswegs auf korneale Schnittfiguren beschränkt und auch nicht auf korneale Merkmale beschränkt.

Die Erfindung wird nachfolgend anhand der beigefügten Zeichnungen weiter erläutert, von denen

Fig. 1 eine Übersichtsdarstellung einer Vorrichtung für ein augenchirurgisches Behandlungssystem gemäß einem Ausführungsbeispiel zeigt,

Fig. 2a eine schematische Darstellung eines von einer Bilderfassungseinheit der in Fig. 1 dargestellten Vorrichtung erzeugten Bildes zeigt, in das mehrere von einer Rechneranordnung der Vorrichtung erzeugte Abbildungen eingeblendet sind,

Fig. 2b eine schematische Darstellung eines von einem Diagnosegerät der in Fig. 1 dargestellten Vorrichtung erzeugten Diagnosebildes zeigt,

Fig. 3 eine Übersichtsdarstellung eines Verfahrens für eine augenchirurgische Behandlung gemäß einem Ausführungsbeispiel zeigt,

Fig. 4 eine weitere Darstellung eines von einem Diagnosegerät der in Fig. 1 dargestellten Vorrichtung erzeugten Diagnosebilds zeigt,

Fig. 5 eine weitere Darstellung eines von einer Bilderfassungseinheit der in Fig. 1 dargestellten Vorrichtung erzeugten Bilds zeigt, in das mehrere von einer Rechneranordnung der Vorrichtung erzeugte Abbildungen eingeblendet sind,

Fig. 6 eine Darstellung eines von einem Diagnosegerät der in Fig. 1 dargestellten Vorrichtung erzeugten Schnittprofils zur Erzeugung eines Diagnosebildes zeigt,

Fig. 7 eine Darstellung eines von einer Bilderfassungseinheit der in Fig. 1 dargestellten Vorrichtung erzeugten Schnittprofils zur Erzeugung eines Bilds zeigt, und

Fig. 8 eine weitere Darstellung eines von einem Diagnosegerät der in Fig. 1 dargestellten Vorrichtung erzeugten Diagnosebilds zeigt.

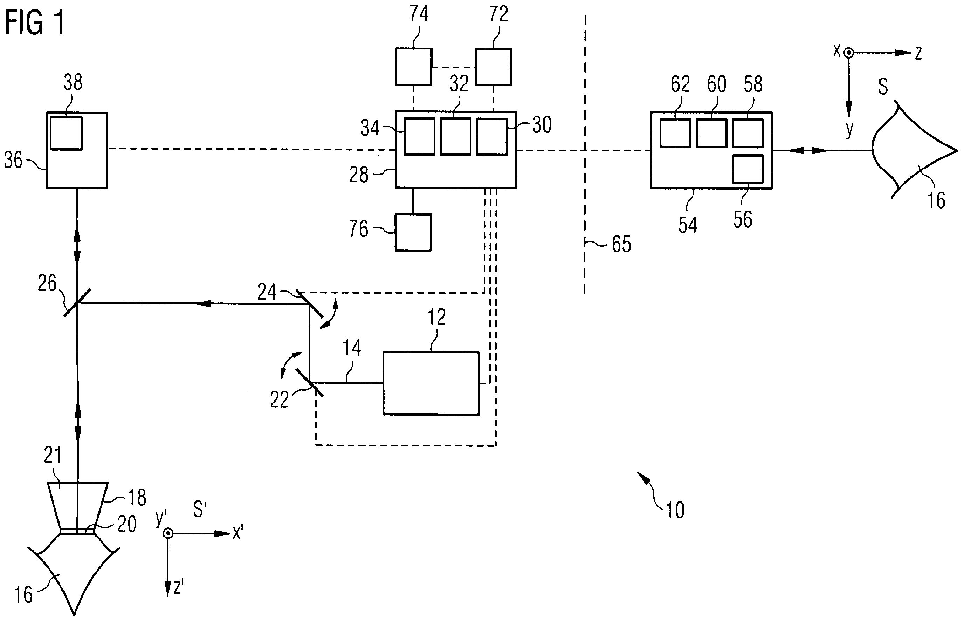

In Fig. 1 sind schematisch Komponenten eines lasergestützten augenchirurgischen Behandlungssystems 10 dargestellt. Dieses Behandlungssystem 10 umfasst einen Laser 12, welcher einen Laserstrahl 14 aus kurzpulsiger Laserstrahlung, beispielsweise mit Pulsdauern im Bereich von Atto-, Femto- oder Pikosekunden, bereitstellt. Der Laserstrahl 14 wird über weiter unten näher beschriebene Mittel zur Strahlführung und -formung auf ein zu behandelndes humanes Auge 16 gerichtet. Das Auge 16 wird mit Hilfe eines Applikators 18 in einem **x',y',z'**-Koordinatensystem S' des Behandlungssystems 10 fixiert. Der Applikator 18 umfasst ein hier beispielhaft als planparallele Applanationsplatte dargestelltes, für die Laserstrahlung transparentes Kontaktelement 20, das beispielsweise gegen das Auge 16 gedrückt wird, so dass sich das Auge 16 mit seiner Vorderfläche an das Kontaktelement 20 anschmiegt. Der Applikator 18 umfasst ferner einen Trägerkörper 21 für das Kontaktelement 20, wobei der Trägerkörper 21 hier beispielhaft als ein sich konisch erweiternder Hülsenkörper dargestellt ist, der im Bereich seines breiteren Hülsenendes lösbar an ein nicht näher dargestelltes Fokussierobjektiv ankoppelbar ist.

Der Laserstrahl 14 wird über mehrere Spiegel 22, 24, 26 in das erwähnte Fokussierobjektiv (beispielsweise ein F-Theta-Objektiv) gerichtet. Im gezeigten Beispielfall sind die Spiegel 22, 24 um zueinander senkrechte Kippachsen schwenkbar, so dass durch entsprechende Ansteuerung der Spiegel 22, 24 der Fokusort des Laserstrahls 14 in der **x',y'**-Ebene (d.h. transversal zur Strahlausbreitungsrichtung am Auge 16) verstellt werden kann. Zur longitudinalen Ortssteuerung des Fokusorts (d.h. in **z'**-Richtung) kann beispielsweise eine längs des Strahlengangs des Laserstrahls 14 verstellbare Linse, eine brechkraftvariable Linse oder ein adaptiv-optischer Spiegel (adaptive optical mirror, ao-mirror) vorgesehen sein (nicht näher dargestellt), mit der/dem sich die Divergenz des Laserstrahls 14 und damit die z'-Position des Strahlfokus beeinflussen lässt. Der Spiegel 26 ist im gezeigten Beispielfall als unbeweglicher, dichroitischer Umlenkspiegel ausgebildet.

Als Steuereinheit des Behandlungssystems 10 dient eine programmgesteuerte Rechneranordnung 28 mit einem Datenspeicher 30, einem Scan-Softwaremodul 32 für die zeitabhängige Ortssteuerung des Strahlungsfokus des Laserstrahls 14 in dem Koordinatensystem S' des Behandlungssystems 10 und einem Bildverarbeitungs-Softwaremodul 34.

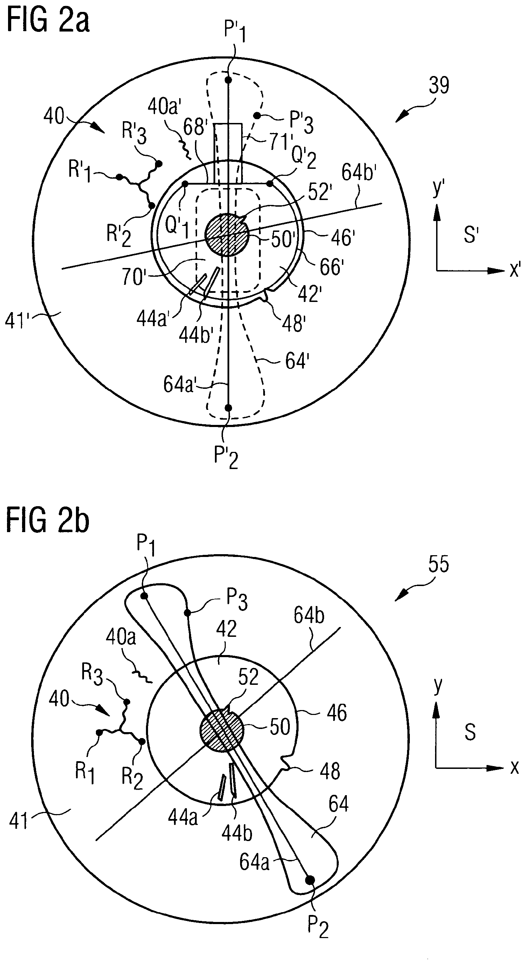

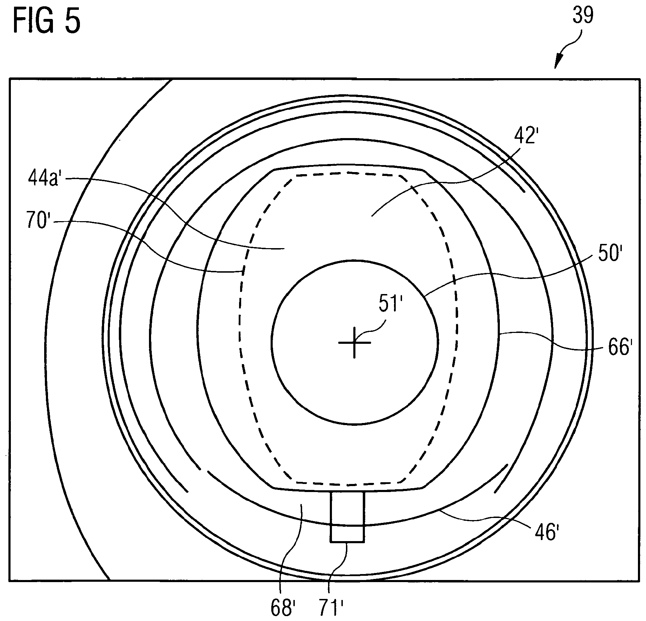

Eine erste Bilderfassungseinheit 36 ist hinter dem dichroitischen Spiegel 26 angeordnet. Bei der Bilderfassungseinheit 36 handelt es sich beispielsweise um eine digitale CCD-Kamera, eine OCT-Bilderfassungseinheit und/oder eine Scheimpflug-Bilderfassungseinheit mit jeweils geeigneter Abbildungsoptik. Der Bilderfassungseinheit 36 ist eine Grünlichtquelle 38 zugeordnet, welche grünes Licht auf das Auge 16 wirft. Die Bilderfassungseinheit 36 erfasst ein zweidimensionales ($x'$-$y'$-Ebene im Koordinatensystem S'), digitales und maßstabsgetreues Bild 39 (vgl. Fig. 2a und Fig. 5) des Auges 16. Das in Fig. 2a und Fig. 5 gezeigte Bild 39 ist eine Draufsicht auf das Auge 16. Das Bild 39 umfasst mindestens eine Abbildung mindestens eines ersten Merkmals des Auges 16. Im Bild 39 in Fig. 2a sind als beispielhafte Merkmale sklerale Blutgefäße 40', 40a' der Sklera 41', die Iris 42' mit Strukturmerkmalen 44a', 44b', der Limbus 46' mit einem Strukturmerkmal 48' und der Pupillenrand 50' mit einem Strukturmerkmal 52' gezeigt. Im Bild 39 in Fig. 5 sind als beispielhafte Merkmale, die Iris 42' mit einem Strukturmerkmal 44a', der Limbus 46' und der Pupillenrand 50' mit dem Pupillenzentrum 51' gezeigt. Im Folgenden wird beispielhaft davon ausgegangen, dass das sklerale Blutgefäß 40' als erstes Merkmal verwendet wird.

Die Bilderfassungseinheit 36 liefert Bilddaten, welche das Bild 39 repräsentieren, an die Rechneranordnung 28. Das Bildverarbeitungs-Softwaremodul 34 verarbeitet diese Bilddaten und wertet sie in noch zu erläuternder Weise aus.

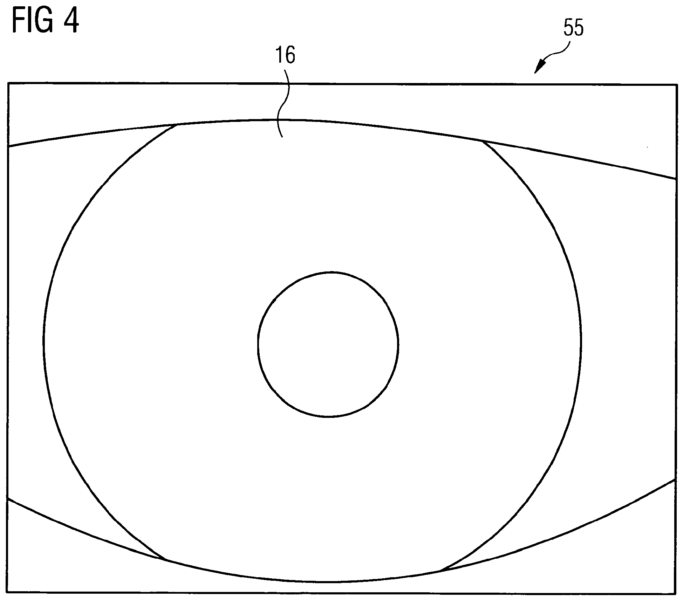

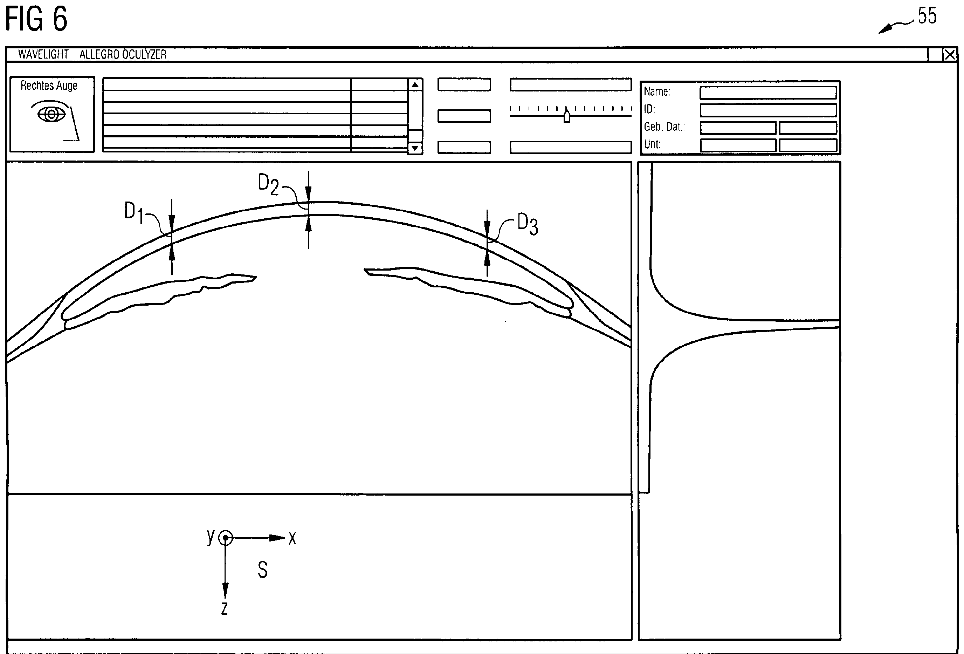

In dem Speicher 30 können diagnostisch ermittelte Referenzdaten vorab gespeichert werden. Zur Ermittlung der Referenzdaten ist im gezeigten Beispielfall der Fig. 1 ein Diagnosegerät 54 vorgesehen, das eine zweite Bilderfassungseinheit 56 umfasst, mittels der in einer zeitlich vor der Laserbehandlung stattfindenden Voruntersuchung des Auges 16 ein zweidimensionales, digitales und maßstabsgetreues Diagnosebild 55 (vgl. Fig. 2b, Fig. 4 und Fig. 8) des zu behandelnden Auges 16 in einem $x,y,z$-Koordinatensystem S des Diagnosegeräts erfasst werden kann. Wie beispielsweise in Fig. 4 und Fig. 6 zu erkennen ist, ist das Auge 16 während der Voruntersuchung nicht durch externe Einwirkung belastet oder deformiert, so dass der Innendruck des Auges 16 seinen natürlichen Wert aufweist. Die Bilderfassungseinheit 56 umfasst beispielsweise eine digitale Kamera sowie einen Topographen (Ophthalmometer, Keratometer oder Videokeratograph), der dazu eingerichtet ist, eine Topographie der Kornea des Auges 16 zu erfassen und daraus jedem Bildpunkt des Diagnosebildes 55 einen Krümmungswert zuzuordnen, der für eine Oberflächenkrümmung der Kornea an einer dem Bildpunkt entsprechenden lateralen Position der Kornea repräsentativ ist. Die von der Kamera und dem Topographen erfassten Daten fließen gemeinsam in das Diagnosebild 55 ein.

Das Diagnosebild 55 gemäß Fig. 2b, Fig. 4 und Fig. 8 ist eine $z$-Draufsicht auf das Auge 16. Auch das Diagnosebild 55 (Fig. 2b) enthält Abbildungen derselben Strukturen, die auch im Bild 39 (Fig. 2a) zu sehen sind. Diese Strukturen sind in Fig. 2b mit gleichen Bezugszeichen bezeichnet wie in Fig. 2a, jedoch ohne Zusatzstrich. Daher enthält das Diagnosebild 55 gemäß Fig. 2b eine Skleraabbildung 41, sklerale Blutgefäßabbildungen 40, 40a, eine Irisabbildung 42 mit Strukturmerkmalen 44a, 44b, eine Limbusabbildung 46 mit einem Strukturmerkmal 48 sowie eine Abbildung des Pupillenrands 50 mit einem Strukturmerkmal 52. Zum Zwecke der besseren Erkennung der augeninternen Merkmale weist das Diagnosegerät 54 eine Grünlichtquelle 58 auf. Ferner umfasst das Diagnosegerät 54 eine Bildverarbeitungseinheit 60, welche zwei bestimmte ausgewählte Merkmale des Auges 16 anhand der von der Bilderfassungseinheit 56 gelieferten Daten erkennen kann. Als erstes Merkmal dient im vorliegenden Beispiel das sklerale Blutgefäß 40. Als zweites Merkmal dient ein astigmatisch gekrümmter Hornhautbereich 64, der durch zwei sich in Fig. 2b in der Pupillenmitte kreuzende Astigmatismusachsen 64a, 64b gekennzeichnet ist.

Die Bildverarbeitungseinheit 60 ist dazu eingerichtet, das erste Merkmal 40 im Diagnosebild 55 durch drei charakteristische, nicht auf einer gemeinsamen Gerade liegende Punkte $R_1$, $R_2$, $R_3$ des ersten Merkmals 40 zu erkennen. Die drei Punkte $R_1$, $R_2$, $R_3$ stellen im vorliegenden Beispiel die Enden von drei sich von einem zentralen Punkt erstreckenden Adern des skleralen Blutgefäßes 40 dar. Die Positionen der Punkte $R_1$, $R_2$, $R_3$ werden in dem Koordinatensystem S des Diagnosegeräts 54 ermittelt und durch drei entsprechende Vektoren $\mathbf{R_1}$, $\mathbf{R_2}$, $\mathbf{R_3}$ eindeutig festgelegt (Vektoren sind hier durch Fettsetzung dargestellt).

Die Position des ersten Merkmals 40 ist im Koordinatensystem S durch die Vektoren $\mathbf{R_1}$, $\mathbf{R_2}$, $\mathbf{R_3}$ eindeutig festgelegt. Ebenso ist die Orientierung des ersten Merkmals 40 im Koordinatensystem S durch die Vektoren $\mathbf{R_1}$, $\mathbf{R_2}$, $\mathbf{R_3}$ oder durch zwei der drei Relativvektoren $\mathbf{R_2}$-$\mathbf{R_1}$, $\mathbf{R_3}$-$\mathbf{R_1}$, $\mathbf{R_3}$-$\mathbf{R_2}$ eindeutig festgelegt. Beispielsweise sind dies die zwei Vektoren $\mathbf{r_{12}}$ und $\mathbf{r_{13}}$, wobei

$$\mathbf{r_{12}} = \mathbf{R_2} - \mathbf{R_1}$$

$$r_{13} = R_3 - R_1$$

Auch die Größe und die Form des ersten Merkmals werden durch die Punkte $R_1$, $R_2$, $R_3$ eindeutig charakterisiert. Da die Punkte $R_1$, $R_2$, $R_3$ nicht auf einer Gerade liegen, sind die Vektoren $r_{12}$ und $r_{13}$ linear unabhängig und spannen im Diagnosebild 55 ein augeninternes und für das Auge 16 individuelles Koordinatensystem auf.

Die Bildverarbeitungseinheit 60 ist ferner dazu eingerichtet, das zweite Merkmal 64 im Diagnosebild 55 durch drei charakteristische, gleichermaßen nicht auf einer Gerade liegende Punkte $P_1$, $P_2$, $P_3$ zu erkennen und diese drei Punkte $P_1$, $P_2$, $P_3$ durch drei entsprechende Vektoren $\mathbf{P_1}$, $\mathbf{P_2}$, $\mathbf{P_3}$ in dem durch die Vektoren $r_{12}$ und $r_{13}$ aufgespannten augeninternen Koordinatensystem darzustellen. Wie in Fig. 2b zu erkennen ist, liegen die Punkte $P_1$ und $P_2$ beispielhaft auf einer der beiden Astigmatismusachsen 64a, 64b. Das Diagnosegerät kann so die Position, die Orientierung, die Größe und die Form des zweiten Merkmals 64 anhand der Bestimmung der Koeffizienten $a_1$, $a_2$, $a_3$, $b_1$, $b_2$, $b_3$ in

$$P_1 = a_1 \times r_{12} + b_1 \times r_{13}$$

$$P_2 = a_2 \times r_{12} + b_2 \times r_{13}$$

$$P_3 = a_3 \times r_{12} + b_3 \times r_{13}$$

ermitteln.

Die Punkte $P_1$, $P_2$, $P_3$ des zweiten Merkmals 64 sind folglich auf das durch das erste Merkmal 40 definierte Koordinatensystem, dessen Ursprung der Punkt $R_1$ bildet, referenziert. Die Koeffizienten $a_1$, $a_2$, $a_3$, $b_1$, $b_2$, $b_3$ sind für das Auge 16 individuell und unabhängig von der Wahl des Koordinatensystems S. Die Koeffizienten $a_1$, $a_2$, $a_3$, $b_1$, $b_2$, $b_3$ können gemeinsam mit digitalen Bilddaten des Diagnosebilds 55 und mit Informationen, aus denen hervorgeht, welches das erste Merkmal 40 ist, auf das das zweite Merkmal 64 referenziert ist, in einer Datenbank 62 als Referenzdaten abgespeichert werden. In Fig. 1 ist die Datenbank 62 in das Diagnosegerät 54 integriert. Die Datenbank kann aber auch geräteunabhängig bzw. geräteextern ausgebildet sein, also etwa als online Datenbank, als mobiler Datenträger (Diskette, CD, DVD, USB-Stick, memory-card, ...) usw.

Für die Laserbehandlung des Auges 16, bei der durch Aneinanderreihung von Photodisruptionen eine Schnittfigur lasertechnisch im Auge 16 erzeugt werden soll, werden die Referenzdaten aus der Datenbank 62 ausgelesen und an die Rechneranordnung 28 übermittelt. Die in Fig. 1 gestrichelte eingezeichnete Trennlinie 65 soll verdeutlichen, dass die Behandlungsstation, an der sich die der Laser 12, die Rechneranordnung 28 und die Bilderfassungseinheit 36 befinden, räumlich getrennt von der Diagnosestation mit dem Diagnosegerät 54 sein kann und dass die Ermittlung der Referenzdaten zeitlich vor der Laserbehandlung des Auges 16 stattfindet.

Das Bildverarbeitungs-Softwaremodul 34 der Rechneranordnung 28 hat Zugriff auf die Datenbank 62, liest die dort gespeicherten Referenzdaten des betreffenden Patienten und ermittelt anhand der Referenzdaten, woran das erste Merkmal 40' zu erkennen ist. Anschließend ermittelt das Bildverarbeitungs-Softwaremodul 34 aus dem von der Bilderfassungseinheit 36 erfassten Bild 39 gemäß Fig. 2a die Positionen der entsprechenden charakteristischen Punkte $R_1'$, $R_2'$, $R_3'$ des ersten Merkmals 40' im Koordinatensystem S' anhand von Koeffizienten $c_1$, $c_2$, $c_3$, $d_1$, $d_2$, $d_3$, wobei folgendes gilt:

$$R_1' = c_1 \times \mathbf{x}' + d_1 \times \mathbf{y}'$$

$$R_2' = c_2 \times \mathbf{x}' + d_2 \times \mathbf{y}'$$

$$R_3' = c_3 \times \mathbf{x}' + d_3 \times \mathbf{y}'$$

Das Koordinatensystem S' wird durch drei Vektoren $\mathbf{x}'$, $\mathbf{y}'$, $\mathbf{z}'$ aufgespannt, wobei $\mathbf{z}'$ parallel zur Richtung des Laserstrahls 14 verläuft und somit im zweidimensionalen Bild 39 nicht erfasst wird. Aus den Koeffizienten $c_1$, $c_2$, $c_3$, $d_1$, $d_2$, $d_3$ ermittelt die Bildverarbeitungseinheit 34 nun die Darstellung von Relativvektoren $r_{12}'$, $r_{13}'$ gemäß

$$r_{12}' = R_2' - R_1'$$

$$r_{13}' = R_3' - R_1'$$

Die Rechneranordnung 28 kann daraus die relativen Positionen der Punkte $P_1'$, $P_2'$, $P_3'$ gegenüber den Punkten $R_1'$, $R_2'$, $R_3'$ mittels

$$P_1' = a_1 \times r_{12}' + b_1 \times r_{13}' + R_1'$$

$$P_2' = a_2 \times r_{12}' + b_2 \times r_{13}' + R_1'$$

$$P_3' = a_3 \times r_{12}' + b_3 \times r_{13}' + R_1'$$

berechnen, wobei dies in Abhängigkeit der vorab durch das Diagnosegerät 54 ermittelten und in den Referenzdaten enthaltenen Koeffizienten $a_1$, $a_2$, $a_3$, $b_1$, $b_2$, $b_3$ erfolgt.

Die Rechneranordnung 28 kann somit die Positionen der das zweite Merkmal 64' charakterisierenden Punkte $P_1$', $P_2$', $P_3$' und damit die Position und die Orientierung des zweiten Merkmals 64' im Koordinatensystem S' des Behandlungssystems 10 ermitteln, ohne dabei das zweite Merkmal 64' selbst in dem von der Bilderfassungseinheit 36 erfassten Bilddaten direkt erkennen zu müssen. Auch die Größe und die Form des zweiten Merkmals 64' im Koordinatensystem S' können automatisch ermittelt werden, da das Bild 39 und das Diagnosebild 55 maßstabsgetreue Abbildungen des Auges 16 sind und anhand der Größe des ersten Merkmals 40, 40' im Bild 39 bzw. im Diagnosebild 55 eine Maßstabsanpassung (Zooming) durch die Rechneranordnung 28 vorgenommen werden kann.

Die Rechneranordnung 28 kann aus der relativen Lage der Vektoren $r_{12}$, $r_{13}$ gegenüber $r_{12}$', $r_{13}$' auch einen Drehwinkel ermitteln, mit dem das Koordinatensystem S gegenüber dem Koordinatensystem S' bezüglich der **z**- bzw. **z**'-Achse verdreht ist. Beliebige Orientierungen des Auges 16 in der **x'** -**y**'-Ebene (etwa durch Verdrehungen des Auges 16 um die **z**'-Achse) können so von der Rechneranordnung 28 erkannt und von dem Behandlungssystem 10 in die Bestimmung der Position Orientierung, Größe und Form der Schnittfigur einbezogen werden, ohne dass dies von einem Arzt oder Chirurgen manuell vorgenommen werden müsste.

Auf Grundlage der für die Positionen $P_1$', $P_2$', $P_3$' und der daraus festgelegten Orientierung $P_1$'-$P_2$', $P_3$'-$P_2$', $P_2$'-$P_1$', Größe und Form des zweiten Merkmals 64' im Koordinatensystems S' berechnet das Scan-Softwaremodul 32 automatisch eine im Auge 16 zu erzeugende Schnittfigur 66'. Die Schnittfigur 66' definiert in gezeigten Beispielfall einen kornealen Flap mit einem durch eine Scharnierachse $Q_1$'-$Q_2$' repräsentierten Scharnier 68' (in der Fachterminologie oft mit dem englischen Begriff "hinge" bezeichnet). Zusätzlich umfasst die Schnittfigur 66' ferner einen Hilfsschnitt 71'.

Der Hilfsschnitt bildet einen Entgasungskanal, durch den Operationsgase, die bei der photodisruptiven Bearbeitung des Augengewebes entstehen, abgeführt werden können. Ein Eindringen solcher Gase in kritische Gewebebereiche des Auges kann so vermieden werden. Es wird bevorzugt zuerst der Hilfsschnitt erzeugt, erst danach wird der Flap geschnitten.

Nach Schneiden des Flaps wird dieser am Hinge 68' hängend zur Seite geklappt, um Korneagewebe (Stroma) freizulegen, welches dann in einem Ablationsgebiet 70' nach Maßgabe eines vorab ermittelten Ablationsprofils mit einem nicht näher dargestellten Excimerlaser des Behandlungssystems 10 bearbeitet wird, um die durch das zweite Merkmal 64' (also den astigmatisch gekrümmten Hornhautbereich) verursachte Sehschwäche des Auges 16 (also den Astigmatismus des Auges 16) zu korrigieren. Die Schnittfigur 66' wird in ihrer Position, Orientierung, Größe und Form durch das Scan-Softwaremodul 32 an die Position, Orientierung, Größe und Form des zweiten Merkmals 64' angepasst. Die Positionen der Punkte $Q_1$', $Q_2$' werden berechnet durch

$$Q_1' = u_1 \times P_1' + v_1 \times P_2',$$

$$Q_2' = u_2 \times P_1' + v_2 \times P_2',$$

$$Q_3' = u_3 \times P_1' + v_3 \times P_2'.$$

Die Koordinaten $u_1$, $u_2$, $u_3$, $v_1$, $v_2$, $v_3$ sind behandlungsspezifisch und an die durch das zweite Merkmal 64' charakterisierte Sehschwäche angepasst. Die Koordinaten $u_1$, $u_2$, $u_3$, $v_1$, $v_2$, $v_3$ werden beispielsweise so angepasst, dass die Scharnierachse $Q_1$'-$Q_2$' des Hinges senkrecht zu der Astigmatismusachse $P_1$'-$P_2$' orientiert ist, die Punkte $Q_1$' und $Q_2$' den gleichen Abstand zur Astigmatismusachse $P_1$'-$P_2$' haben und bezüglich ihrer lateralen Positionen (also entlang **x**' und **y**' im Koordinatensystem S') in einem dem Limbus 46' angenäherten Bereich der Iris 42' angeordnet sind, siehe Figur 2a.

Der Position, Orientierung, Form und/oder Größe des Hilfsschnitts 71' sind behandlungsspezifisch und an die Position und Orientierung des Hinges 68', insbesondere an die Position und Orientierung der Scharnierachse $Q_1$'-$Q_2$' angepasst. Der mit Hilfsschnitt 71' erstreckt sich von der Kornea des Auges 16 bis zur Sklera 41' des Auges 16 und durchläuft dabei den Limbus 46'. Der flächig, im Wesentlichen als ebener Kanal ausgebildete Hilfsschnitt 71' steht mit der restlichen Schnittfigur 66' in Verbindung und endet an der Oberfläche des Auges 16. Der Hilfsschnitt 71' ermöglicht es daher, dass beim Schneiden des Flaps und der restlichen Schnittfigur 66' entstehende Gase aus dem Auge 16 entweichen können.

Die Rechneranordnung 28 ist dazu eingerichtet, eine bildliche Darstellung der Schnittfigur 66' und des zweiten Merkmals 64' sowie ggf. des Ablationsgebiets 70' nach Maßgabe der für diese Elemente ermittelten Position und Orientierung sowie Größe und Form im Koordinatensystem S' zu erzeugen. Das Behandlungssystem 10 kann eine Vorrichtung 72 umfassen, die dazu eingerichtet ist, diese bildliche Darstellung dem von der Bilderfassungseinheit 36 erfassten Bild 39 zu überlagern und das daraus entstandene Gesamtbild - so wie in Fig. 2a dargestellt - auf einem Ausgabegerät 74 (z.B. Monitor) maßstabsgetreu anzuzeigen. Ein derartiges Gesamtbild ist in Fig. 5 dargestellt. Alternativ oder zusätzlich kann die Vorrichtung 72 die bildliche Darstellung in das Beobachtungsbild eines OP-Mikroskops des Behandlungssystems 10 (nicht näher dargestellt) einblenden.

Die von der Rechneranordnung 28 ermittelte Position, Orientierung und Dimensionierung des Schnittbilds 66' kann so von dem behandelnden Arzt oder Chirurgen in Relation zum ersten Merkmal 40' des Auges 16 beobachtet und kontrolliert werden.

Die Schnittfigur 66' dient als ein von der Rechneranordnung 28 ermittelter Vorschlag, der modifiziert werden kann. Ist der Arzt/Chirurg mit dem Vorschlag unzufrieden, kann er die Position, Orientierung und Dimensionierung der Schnittfigur 66' abändern, um die Behandlung noch idealer zu gestalten. Hierzu kann sich der Arzt/Chirurg einer Eingabevorrichtung 76 des Behandlungssystems 10 bedienen, die es ihm erlaubt, gewünschte Modifikationen der Position, Orientierung, Größe oder/und Form der Schnittfigur 66' per manuelle Eingabe an die Rechneranordnung 28 zu übermitteln. Die Rechneranordnung 28 berücksichtigt diese Modifikationen und ermittelt entsprechend die Position, Orientierung, Größe oder/und Form der Schnittfigur 66' neu. Da die angezeigte/eingeblendete Visualisierung der Schnittfigur 66' stets die aktuelle Position, Orientierung, Größe und Form der Schnittfigur 66' relativ zum zweiten Merkmal 64' im Bild 39 wiederspiegelt, kann diese Optimierung durch den Arzt/Chirurgen sozusagen online erfolgen.

Sobald der Arzt/Chirurg mit der Position, Orientierung, Größe und Form der Schnittfigur 66' bzw. der visualisierten Darstellung derselben zufrieden ist, kann er die aktuelle Schnittfigur 66' per Eingabe über die Eingabevorrichtung 76 manuell bestätigen. Anschließend erzeugt das Behandlungssystem 10 dann die durch den Arzt/Chirurgen bestätigte Schnittfigur 66' mittels des Lasers 12 im Auge 16 des Patienten.

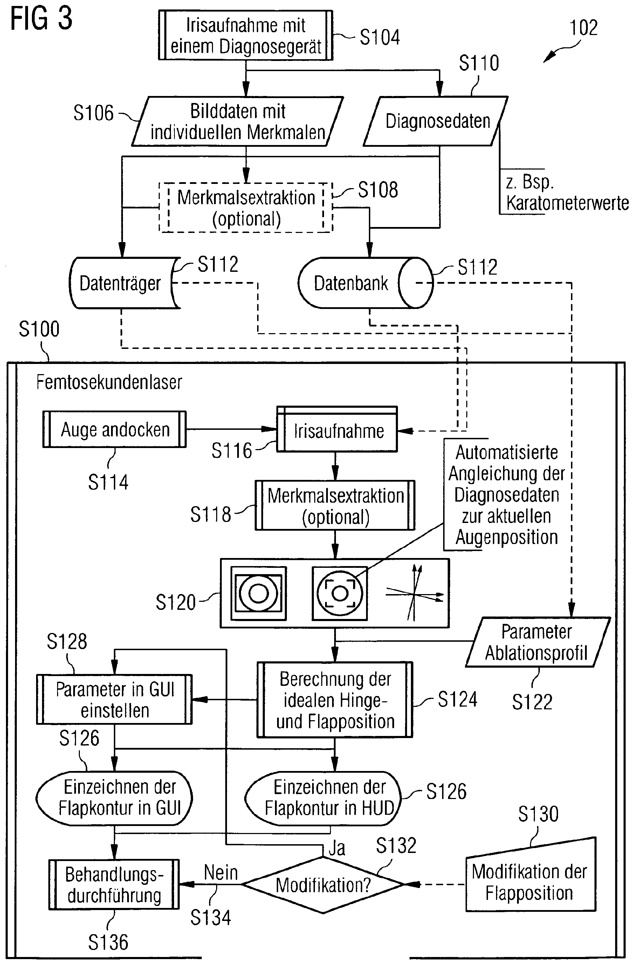

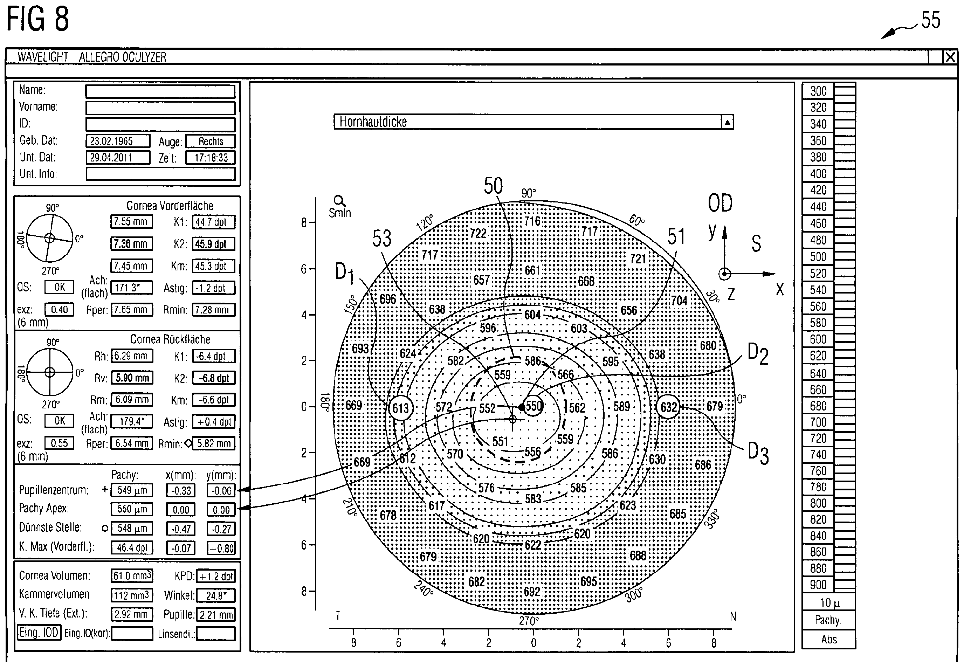

In Figur 3 ist noch einmal die eben beschriebene Behandlungsprozedur in Form eines Flussdiagramms dargestellt. Zunächst wird in einer zeitlich vor der Laserbehandlung S100 stattfindenden Voruntersuchung S102 des Auges 16 eine das Diagnosebild 55 darstellende Irisaufnahme (vgl. auch Fig. 2b und Fig. 4) des zu behandelnden Auges 16 erfasst, siehe Schritt S104. Aus der Irisaufnahme werden Bilddaten erzeugt, die individuelle Merkmale 40-52 des Auges 16, siehe Schritt S106. Im Rahmen einer Merkmalsextraktion S108 werden Position, Orientierung und Größe des ersten Merkmals 40 in dem $x,y,z$-Koordinatensystem S des Diagnosegeräts 54 ermittelt. Außerdem werden parallel Diagnosedaten ermittelt, siehe Schritt S110. Die Diagnosedaten umfassen insbesondere Keratometerwerte, eine korneale Dickenverteilung (wie in Fig. 8 dargestellt) sowie Position, Orientierung und Größe (Länge) der Astigmatismusachsen 64a, 64b (wie in Fig. 2b dargestellt) im Koordinatensystem S. Die während der Voruntersuchung S102 ermittelten Daten werden in einer Datenbank oder auf einem Datenträger gespeichert, siehe Schritt S112.

Nach Abschluss der Voruntersuchung S102 findet die eigentliche Laserbehandlung S100 statt. Hierzu wird im Schritt S114 das zu behandelnde Auge 16 an die Applanationslinse 20 des Behandlungssystems 10 angedockt und im Schritt 116 ein Bild 39 des Auges 16 aufgenommen. Im Rahmen einer Merkmalsextraktion S118 werden Position, Orientierung und Größe des ersten Merkmals 40' in dem $x',y',z'$-Koordinatensystem S' des Behandlungssystem 10 ermittelt. Durch Abgleich der so ermittelten Position, Orientierung und Größe des ersten Merkmals 40' im Koordinatensystem S' mit der aus dem Datenspeicher bzw. der Datdank ausgelesenen Position, Orientierung und Größe des ersten Merkmals 40 im Koordinatensystem S wird die Position, Orientierung und Größe (Länge) der Astigmatismusachsen 64a', 64b' im Koordinatensystem S' rechnerisch ermittelt.

Im Schritt S122 werden anhand der vorab ermittelten Diagnosedaten, insbesondere der Keratometerwerte, Parameter für ein Ablationsprofil errechnet, die an die Position, Orientierung und Größe (Länge) der Astigmatismusachsen 64a', 64b' im Koordinatensystem S' angepasst sind. Um dieses Ablationsprofil im Auge 16 zur Bearbeitung auch tatsächlich freilegen zu können, werden im Schritt S124 die Position, die Orientierung, die Form und die Größe der Schnittfigur 66' einschließlich der entsprechenden Positionen, Orientierungen, Formen und Größen des Flaps, des Hinges und des Hilfsschnitts 71' berechnet. Die so berechnete Schnittfigur 66' wird im Schritt S126 zusammen mit der im Schritt S116 erfassten Aufnahme auf einer graphischen Benutzeroberfläche (graphical user interface, GUI) angezeigt oder/und durch eine geeignete Einblendeinrichtung (nach Art eines Head-Up-Displays, HUD) in den Beobachtungsstrahlengang des Operationsmikroskops eingeblendet (siehe auch Fig. 2b und Fig. 5).

Position, Orientierung, Form und Größe der Schnittfigur 66' können von einem die Behandlung leitenden Operateur modifiziert werden, siehe Schritt S130. Bei einer Modifikation S132 ändert der Operateur die vom Behandlungssystem vorgeschlagenen Parameter durch manuelles Einstellen über das GUI. Danach wird die Schnittfigur 66' gemäß der Modifikation neu ermittelt und auf dem GUI bzw. im HUD neu dargestellt. Sobald der Operateur mit der Position, Orientierung, Form und Größe der Schnittfigur 66' zufrieden ist, bestätigt er die eingestellten Parameter im Schritt S134. Hiernach wird die Behandlung des Auges 16 entsprechende der eingestellten Parameter vom Behandlungssystem 10 vorgenommen, siehe Schritt S136. Alternativ ist auch denkbar, dass das zweite Merkmal einen krankhaften Gewebebereich des Auges 16 eines Patienten darstellt, der die Sicht des Patienten trübt, wie zum Beispiel ein Katarakt-Bereich, also ein Bereich, der an Grauem Star erkrankt ist. Die Schnittfigur 66' ist dann in ihrer Position, Orientierung, Größe und Form mit Bezug auf den Katarakt-Bereich zu ermitteln und in der humanen Linse des Auges 16 zu bewirken.

Das Diagnosegerät 54 umfasst beispielsweise eine digitale Kamera sowie einen Topographen (Ophthalmometer, Keratometer oder Videokeratograph) und ist dazu eingerichtet, eine Topographie der Kornea des Auges 16 und/oder eine korneale Dickenverteilung des Auges 16 zu erfassen und daraus jedem Bildpunkt des Diagnosebildes 55 einen Krümmungswert zuzuordnen, der für eine Oberflächenkrümmung der Kornea an einer dem Bildpunkt entsprechenden lateralen Position der Kornea repräsentativ ist.

Das Diagnosegerät 54 kann ferner dazu eingerichtet sein, im Rahmen einer pachymetrischen Aufnahme des Auges 16 eine korneale Dickenverteilung des Auges 16 zu erfassen, siehe Fig. 8. Hierbei wird jedem Bildpunkt des Diagnosebildes 55 ein Dickenwert zugeordnet, der für die Dicke der Kornea an einer dem Bildpunkt entsprechenden lateralen Position der Kornea repräsentativ ist. In dieser Darstellung ist der Pupillenrand 50, das Pupillenzentrum 51 und der korneale Apex 53 zu erkennen. Die verschiedenen Dickenwerte bzw. die sich aus den Dickenwerten ergebene korneale Dickenverteilung erlauben eine individuelle Charakterisierung des Auges. Die korneale Dickenverteilung kann daher als das erste Merkmal dienen. Die korneale Dickenverteilung definiert dann ein augeninternes Koordinatensystem auf das Position, Orientierung und Größe des zweiten Merkmals, etwa ein astigmatisch verformter Hornhautbereich oder Astigmatismusachsen, referenziert werden. Hierzu kann beispielsweise der korneale Apex 53 als Koordinatenursprung (x[mm]=0, y=[mm]) gewählt werden.

Die korneale Dickenverteilung wird beispielsweise anhand einer OCT-Messung oder einer Scheimpflug-Messung ermittelt. Im Falle einer OCT-Messung wird eine Mehrzahl von zweidimensionalen Schnittprofilen des Auges 16 erfasst, anhand derer zweidimensionale und/oder dreidimensionale Abbildungen des Auges 16 möglich sind. Zum Beispiel verlaufen hierzu die Schnittprofile parallel zueinander oder schneiden sich gegenseitig entlang einer Augenachse (Sehachse, optische Achse des Auges, ...). Ein derartiges Schnittprofil ist in Fig. 6 zu sehen. Das Diagnosegerät 54 ermittelt aus den Schnittprofilen jeweils die Dickenwerte der Kornea entlang des dem jeweiligen Schnittprofil entsprechenden Querschnitts. Exemplarisch sind drei Dickenwerte $D_1$, $D_2$, $D_3$ der Kornea in Fig. 6 gekennzeichnet. Die Gesamtheit der Dickenwerte ergibt in ihrer räumlichen Zuordnung die korneale Dickenverteilung, wie in Fig. 8 dargestellt. Bereiche konstanter Dicke erscheinen als Höhenlinien. Zur besseren Unterscheidung der verschiedenen Höhenlinien sind selbige farblich codiert. Aus einem OCT-Bild können auch charakteristische Schichtenverteilungen extrahiert werden.

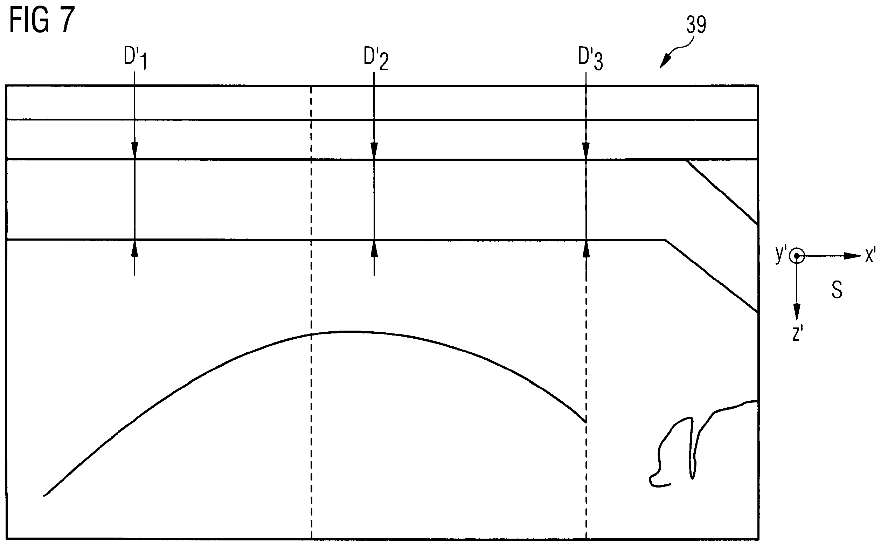

Ebenso wie das Diagnosegerät 54 kann das Behandlungssystem 10 dazu eingerichtet sein, im Rahmen einer pachymetrischen Aufnahme des Auges 16 die korneale Dickenverteilung des Auges 16 zu erfassen. In Fig. 7 ist eine OCT-Aufnahme dargestellt, die ein Schnittprofil der Kornea bei der Behandlung zeigt und aus der Dickenwerte für die korneale Dickenverteilung gewonnen werden. Die Kornea befindet sich dabei in einem eingeebneten Zustand, der mit Hilfe der Applanationslinse 20 bewirkt wird. Exemplarisch sind in Fig. 7 drei, den in Fig. 6 gezeigten Dickenwerten $D_1$, $D_2$, $D_3$ entsprechende Dickenwerte $D_1'$, $D_2'$, $D_3'$ der Kornea gekennzeichnet.

Da die korneale Dickenverteilung sowohl im applanierten als auch im entspannten Zustand unverändert bleibt, behält die während der Voruntersuchung mit Hilfe des Diagnosegeräts 54 ermittelte Referenzierung des astigmatisch verformten Hornhautbereichs bzw. der Astigmatismusachsen während der eigentlichen Behandlung seine Gültigkeit.

**Patentansprüche**

1. Vorrichtung für ein lasergestütztes augenchirurgisches Behandlungssystem (10), umfassend:

- eine erste Bilderfassungseinheit (36), die dazu eingerichtet ist, ein erstes Bild (39) eines zu behandelnden Auges (16) zu erfassen,
- eine Rechneranordnung (28), die dazu eingerichtet ist, durch Bildverarbeitung des ersten Bilds mindestens ein erstes Merkmal (40, 40') des Auges (16) zu erkennen und eine Position und eine Orientierung des ersten Merkmals in einem Koordinatensystem (S') des Behandlungssystems (10) zu ermitteln, wobei die Rechneranordnung ferner dazu eingerichtet ist, eine Position und eine Orientierung einer in dem Auge (16) zu erzeugenden Schnittfigur (66') in dem Koordinatensystem (S') des Behandlungssystems (10) abhängig von der ermittelten Position und Orientierung des ersten Merkmals in dem Koordinatensystem sowie abhängig von einer vorab ermittelten relativen Position und Orientierung mindestens eines zweiten Merkmals (64, 64') des Auges (16) gegenüber dem ersten Merkmal (40, 40') zu ermitteln,

dadurch gekennzeichnet, dass die Schnittfigur (66') einen kornealen Flap mit einem Flapscharnier (68') definiert, wobei die Rechneranordnung (28) dazu eingerichtet ist, die Position und Orientierung eines das Flapscharnier (68') definierenden Figurelements der Schnittfigur abhängig von der Position und Orientierung des ersten Merkmals (40, 40') in dem Koordinatensystem (S') des Behandlungssystems (10) sowie abhängig von der relativen Position und Orientierung des zweiten Merkmals (64, 64') gegenüber dem ersten Merkmal (40, 40') zu ermitteln, dass das mindestens eine zweite Merkmal (64, 64') einen astigmatisch gekrümmten Hornhautbereich umfasst und die Rechneranordnung (28) dazu eingerichtet ist, die Position und Orientierung der Schnittfigur (66') unter Berücksichtigung einer vorgegebenen Soll-Lagebedingung zwischen dem Flap und dem astigmatisch gekrümmten Hornhautbereich zu ermitteln, und/oder dass dem Flapscharnier (68') eine Scharnierachse ($Q_1'$-$Q_2'$) zugeordnet ist und dem astigmatisch gekrümmten Hornhautbereich eine Astigmatismusachse ($P_1'$-$P_2'$) zugeordnet ist, wobei die Rechneranordnung (28) dazu eingerichtet ist, die Position und Orientierung der Schnittfigur (66') unter Berücksichtigung einer vorgegebenen Soll-Lagebedingung zwischen der Scharnierachse und der Astigmatismusachse zu ermitteln, wobei insbesondere die Soll-Lagebedingung zwischen der Scharnierachse ($Q_1$'-$Q_2$') und der Astigmatismusachse ($P_1$'-$P_2$') eine zueinander im wesentlichen senkrechte Lage der beiden Achsen vorgibt.

2. Vorrichtung nach einem der vorhergehenden Ansprüche, wobei das mindestens eine erste Merkmal (40, 40') einer Iris, einer Pupille, einem Limbus, einer skleralen Blutgefäßanordnung oder/und einer kornealen Dickenverteilung des Auges (16) zugeordnet ist.

3. Vorrichtung nach einem der vorhergehenden Ansprüche, umfassend ein Diagnosegerät (54) mit einer zweiten Bilderfassungseinheit (56) zur Erfassung eines zweiten Bilds (55) des zu behandelnden Auges (16), wobei das Diagnosegerät (54) dazu eingerichtet ist, in dem zweiten Bild durch Bildverarbeitung das mindestens eine erste Merkmal (40, 40') zu erkennen und Merkmalsinformationen betreffend eine Position und Orientierung jedes der beiden Merkmale zu erzeugen wobei insbesondere:

   - die Rechneranordnung (28) dazu eingerichtet ist, auf Grundlage der Merkmalsinformationen die relative Position und Orientierung des zweiten Merkmals (64, 64') gegenüber dem ersten Merkmal (40, 40') zu ermitteln, und/oder
   - das Diagnosegerät (54) und die erste Bilderfassungseinheit (36) verschiedenen Arbeitsstationen in einer ärztlichen Praxis zugeordnet sind, und/oder
   - dem Diagnosegerät (54) eine Datenbank (62) zugeordnet ist, um darin die Merkmalsinformationen oder/und hiervon abgeleitete Informationen in Zuordnung zu Patientenkenninformationen abzulegen, und wobei die Rechneranordnung (28) Zugang zu der Datenbank besitzt.

4. Vorrichtung nach einem der vorhergehenden Ansprüche, wobei die Rechneranordnung (28) dazu eingerichtet ist, eine bildliche Darstellung der Schnittfigur (66') zu bewirken, welche die ermittelte Position und Orientierung der Schnittfigur in Relation zu dem ersten Merkmal (40. 40') oder/und dem zweiten Merkmal (64, 64') oder/und einem zu ablatierenden Korneabereich (70') veranschaulicht wobei insbesondere:

   - die Rechneranordnung (28) dazu eingerichtet ist, die bildliche Darstellung auf einem Monitor oder/und durch Einblendung in einen Beobachtungsstrahlengang eines Operationsmikroskops zu bewirken, und/oder
   - die Rechneranordnung (28) dazu eingerichtet ist, die ermittelte Position oder/und Orientierung der Schnittfigur (66') nach Maßgabe einer Benutzereingabe zu modifizieren und die bildliche Darstellung der Schnittfigur gemäß der modifizierten Position oder/und Orientierung zu modifizieren.

5. Vorrichtung nach einem der vorhergehenden Ansprüche, wobei die Rechneranordnung (28) dazu eingerichtet ist, eine benutzerseitig eingegebene Bestätigung für die Position und Orientierung der Schnittfigur (66') zu empfangen und abhängig vom Empfang dieser Bestätigung Steuerdaten für eine Lasereinrichtung (12) zu erzeugen und die Lasereinrichtung nach Maßgabe dieser Steuerdaten zur Erzeugung der Schnittfigur in dem Auge zu steuern.

6. Verfahren für eine augenchirurgische Behandlung, umfassend:

   - Erfassen eines ersten Bilds eines zu behandelnden Auges,
   - Auswerten des ersten Bilds zur Erkennung mindestens eines ersten Merkmals des Auges,
   - Ermitteln einer Position und einer Orientierung des ersten Merkmals in einem Koordinatensystem eines lasergestützten Augenbehandlungssystems,
   - Ermitteln einer Position und einer Orientierung einer in dem Auge zu erzeugenden Schnittfigur in dem Koordinatensystem des Behandlungssystems abhängig von der ermittelten Position und Orientierung des ersten Merkmals in dem Koordinatensystem sowie abhängig von einer vorab ermittelten relativen Position und Orientierung mindestens eines zweiten Merkmals des Auges gegenüber dem ersten Merkmal,

   **dadurch gekennzeichnet, dass** die Schnittfigur einen kornealen Flap mit einem Flapscharnier definiert und das Verfahren umfasst: Ermitteln der Position und Orientierung eines das Flapscharnier definierenden Figurelements der Schnittfigur abhängig von der ermittelten Position und Orientierung des ersten Merkmals in dem Koordinatensystem des Behandlungssystems sowie abhängig von der relativen Position und Orientierung des ersten Merkmals gegenüber dem zweiten Merkmal, dass das mindestens eine zweite Merkmal einen astigmatisch gekrümmten Hornhautbereich umfasst und das Verfahren umfasst: Ermitteln der Position und Orientierung der Schnittfigur unter Berücksichtigung einer vorgegebenen Soll-Lagebedingung zwischen dem Flap und dem astigmatisch gekrümmten Hornhautbereich, und/oder

   dass dem Flapscharnier eine Scharnierachse zugeordnet ist und dem astigmatisch gekrümmten Hornhautbereich eine Astigmatismusachse zugeordnet ist, wobei das Verfahren umfasst: Ermitteln der Position und Orientierung der Schnittfigur unter Berücksichtigung einer vorgegebenen Soll-Lagebedingung zwischen der Scharnierachse und der Astigmatismusachse, wobei insbesondere die Soll-Lagebedingung zwischen der Scharnierachse und der Astigmatismusachse eine zueinander im wesentlichen senkrechte Lage der beiden Achsen vorgibt.

7.  Verfahren nach Anspruch 6, wobei das mindestens eine erste Merkmal einer Iris, einer Pupille, einem Limbus, einer skleralen Blutgefäßanordnung oder/und einer kornealen Dickenverteilung des Auges zugeordnet ist.

8.  Verfahren nach Anspruch 6, umfassend: Erfassen eines zweiten Bilds des zu behandelnden Auges, Erkennen des mindestens einen ersten Merkmals in dem zweiten Bild durch Bildverarbeitung, und Ermitteln von Merkmalsinformationen betreffend eine Position und Orientierung jedes der beiden Merkmale, wobei das Verfahren ferner insbesondere umfasst:

   - Ermitteln der relativen Position und Orientierung des zweiten Merkmals gegenüber dem ersten Merkmal auf Grundlage der Merkmalsinformationen, und/oder
   - Bereitstellen gesonderter Kamerasysteme zur Erfassung des ersten Bilds und des zweiten Bilds, wobei die Kamerasysteme insbesondere verschiedenen Arbeitsstationen in einer ärztlichen Praxis zugeordnet sind, und/oder
   - Abspeichern der Merkmalsinformationen oder/und hiervon abgeleiteter Informationen in einer Datenbank in Zuordnung zu Patientenkenninformationen.

9.  Verfahren nach Anspruch 6, umfassend: Erzeugen einer bildlichen Darstellung der Schnittfigur, welche die ermittelte Position und Orientierung der Schnittfigur in Relation zu dem ersten Merkmal oder/und dem zweiten Merkmal oder/und einem zu ablatierenden Korneabereich veranschaulicht, wobei das Verfahren ferner insbesondere umfasst:

   - Ausgeben der bildlichen Darstellung auf einem Monitor, und/oder
   - Einblenden der bildlichen Darstellung in einen Beobachtungsstrahlengang eines Operationsmikroskops, und/oder
   - Modifizieren der ermittelten Position oder/und Orientierung der Schnittfigur nach Maßgabe einer Benutzereingabe und Modifizieren der bildlichen Darstellung der Schnittfigur gemäß der modifizierten Position oder/und Orientierung derselben.

10. Verfahren nach Anspruch 6, umfassend: Empfangen einer benutzerseitig eingegebenen Bestätigung für die Position und Orientierung der Schnittfigur, Erzeugen von Steuerdaten für eine Lasereinrichtung abhängig vom Empfang dieser Bestätigung, und Steuern der Lasereinrichtung nach Maßgabe der Steuerdaten zur Erzeugung der Schnittfigur in dem Auge.

11. Vorrichtung nach einem der Ansprüche 1 bis 5, wobei die Schnittfigur ferner einen Hilfskanal definiert, welcher sich von einer Schnittfläche des Flaps in Richtung weg von dem Flap erstreckt, wobei die Rechneranordnung (28) dazu eingerichtet ist, abhängig von der ermittelten Position und Orientierung zumindest solcher Teile der Schnittfigur, die den Flap definieren, Steuerdaten für die Erzeugung des Hilfskanals derart zu erzeugen, dass sich der Hilfskanal bis mindestens in den Bereich des Limbus des Auges erstreckt, wobei die Rechneranordnung (28) insbesondere dazu eingerichtet ist, die Steuerdaten für die Erzeugung des Hilfskanals derart zu erzeugen, dass sich der Hilfskanal bis jenseits des Limbus des Auges erstreckt.

12. Verfahren nach einem der Ansprüche 6 bis 10, wobei die Schnittfigur ferner einen Hilfskanal definiert, welcher sich von einer Schnittfläche des Flaps in Richtung weg von dem Flap erstreckt, wobei das Verfahren umfasst: derartiges Erzeugen von Steuerdaten für die Erzeugung des Hilfskanals abhängig von der ermittelten Position und Orientierung zumindest solcher Teile der Schnittfigur, die den Flap definieren, dass sich der Hilfskanal bis mindestens in den Bereich des Limbus des Auges erstreckt, wobei das Verfahren insbesondere umfasst: Erzeugen der Steuerdaten für die Erzeugung des Hilfskanals derart, dass sich der Hilfskanal bis jenseits des Limbus des Auges erstreckt.

## Claims

1.  Apparatus for a laser-assisted ophthalmic surgical treatment system (10), comprising:

   - a first image capturing unit (36), configured to capture a first image (39) of an eye (16) to be treated,
   - a computer arrangement (28), configured to identify at least a first feature (40, 40') of the eye (16) by image processing of the first image and to ascertain a position and orientation of the first feature in a coordinate system (S') of the treatment system (10), wherein the computer arrangement is further configured to ascertain the position and orientation of an incision figure (66'), to be produced in the eye (16), in the coordinate system (S') of the treatment system (10) as a function of the ascertained position and orientation of the first feature in the coordinate system and as a function of a relative position and orientation, ascertained in advance, of at least one second feature (64, 64') of the eye (16) in relation to the first feature (40, 40'),

**characterized in that** the incision figure (66') defines a corneal flap with a flap hinge (68'), wherein the computer arrangement (28) configured to ascertain the position and orientation of a figure element of the incision figure, defining the flap hinge (68'), as a function of the position and orientation of the first feature (40, 40') in the coordinate system (S') of the treatment system (10) and as a function of the relative position and orientation of the second feature (64, 64') in relation to the first feature (40, 40'),

**in that** the at least one second feature (64, 64') comprises a corneal region with astigmatic curvature and the computer arrangement (28) is configured to ascertain the position and orientation of the incision figure (66') taking account of a predetermined desired relative orientation stipulation between the flap and the corneal region with astigmatic curvature, and/or

**in that** a hinge axis ($Q_1'$-$Q_2'$) is assigned to the flap hinge (68') and an astigmatism axis ($P_1'$-$P_2'$) is assigned to the corneal region with astigmatic curvature, wherein the computer arrangement (28) is configured to ascertain the position and orientation of the incision figure (66') taking account of a predetermined desired relative orientation stipulation between the hinge axis and the astigmatism axis, wherein, in particular, the desired relative orientation stipulation between the hinge axis ($Q_1'$-$Q_2'$) and the astigmatism axis ($P_1'$-$P_2'$) predetermines a relative orientation of the two axes that are substantially perpendicular to one another.

2. Apparatus according to the preceding claim, wherein the at least one first feature (40, 40') is assigned to an iris, a pupil, a limbus, a scleral blood vessel arrangement or/and a corneal thickness distribution of the eye (16).

3. Apparatus according to either of the preceding claims, comprising a diagnostic appliance (54) with a second image capturing unit (56) for capturing a second image (55) of the eye (16) to be treated, wherein the diagnostic appliance (54) is configured to identify the at least one first feature (40, 40') in the second image by image processing and to produce feature information relating to a position and orientation of each of the two features, wherein more particularly:

   - the computer arrangement (28) is configured to ascertain the relative position and orientation of the second feature (64, 64') in relation to the first feature (40, 40') on the basis of the feature information and/or
   - the diagnostic appliance (54) and the first image capturing unit (36) are assigned to different workstations in a physician's practice, and/or
   - a database (62) is assigned to the diagnostic appliance (54) in order to store the feature information or/and information derived therefrom in a manner associated with patient characteristic information in said database, and wherein the computer arrangement (28) has access to the database.

4. Apparatus according to any one of the preceding claims, wherein the computer arrangement (28) is configured to bring about a pictorial representation of the incision figure (66') which elucidates the ascertained position and orientation of the incision figure in relation to the first feature (40, 40') or/and the second feature (64, 64') or/and a corneal region (70') to be ablated, wherein more particularly:

   - the computer arrangement (28) is configured to bring about the pictorial representation on a monitor or/and by superposition into an observation beam path of a surgical microscope, and/or
   - the computer arrangement (28) is configured to modify the ascertained position or/and orientation of the incision figure (66') according to a stipulation of a user entry and to modify the pictorial representation of the incision figure in accordance with the modified position or/and orientation.

5. Apparatus according to any one of the preceding claims, wherein the computer arrangement (28) is configured to receive a confirmation, entered on part of the user, for the position and orientation of the incision figure (66') and to produce control data for a laser device (12) depending on the reception of this confirmation and to control the laser device according to the stipulation of these control data for the purposes of producing the incision figure in the eye.

6. Method for an ophthalmic surgical treatment, comprising:

   - capturing the first image of an item treated,
   - evaluating the first image for identifying at least one first feature of the eye,
   - ascertaining a position and an orientation of the first feature in a coordinate system of a laser-assisted eye treatment system,
   - ascertaining a position and an orientation of an incision figure, to be produced in the eye, in the coordinate system of the treatment system as a function of the ascertained position and orientation of the first feature in the coordinate system and as a function of a relative position and orientation, ascertained in advance, of at least one second feature of the eye in relation to the first feature,

**characterized in that** the incision figure defines a corneal flap with a flap hinge and the method comprises: ascertaining the position and orientation of a figure element of the incision figure, defining the flap hinge, as a function of the ascertained position and orientation of the first feature in the coordinate system of the treatment system and as a function of the relative position and orientation of the first feature in relation to the second feature,

**in that** the at least one second feature comprises a corneal region with astigmatic curvature and the method comprises: ascertaining the position and orientation of the incision figure taking account of a predetermined desired relative orientation stipulation between the flap and the corneal region with astigmatic curvature, and/or

**in that** a hinge axis is assigned to the flap hinge and an astigmatism axis is assigned to the corneal region with astigmatic curvature, wherein the method comprises: ascertaining the position and orientation of the incision figure taking account of a predetermined desired relative orientation stipulation between the hinge axis and the astigmatism axis, wherein more particularly the desired relative orientation stipulation between the hinge axis and the astigmatism axis predetermines a relative orientation of the two axes that are substantially perpendicular to one another.

7. Method according to Claim 6, wherein the at least one first feature is assigned to an iris, a pupil, a limbus, a scleral blood vessel arrangement or/and a corneal thickness distribution of the eye.

8. Method according to Claim 6, comprising: capturing a second image of the eye to be treated, identifying the at least one first feature in the second image by image processing, and ascertaining feature information relating to a position and orientation of each of the two features, wherein the method further more particularly comprises:

   - ascertaining the relative position and orientation of the second feature in relation to the first feature on the basis of the feature information, and/or
   - providing separate camera systems for capturing the first image and the second image, wherein the camera systems more particularly are assigned to different workstations in a physician's practice, and/or

   - storing the feature information and/are information derived therefrom in a database, in a manner associated with patient characteristic information.

9. Method according to Claim 6, comprising: producing a pictorial representation of the incision figure, which elucidates the ascertained position and orientation of the incision figure in relation to the first feature or/and the second feature or/and a corneal region to be ablated, wherein the method furthermore particularly comprises:

   - outputting the pictorial representation on a monitor, and/or
   - superposing the pictorial representation into an observation beam path of a surgical microscope, and/or
   - modifying the ascertained position or/and orientation of the incision figure according to the stipulation of the user entry and modifying the pictorial representation of the incision figure in accordance with the modified position or/and orientation of same.

10. Method according to Claim 6, comprising: receiving a confirmation, entered on part of the user, for the position and orientation of the incision figure, producing control data for a laser device depending on the reception of this confirmation, and controlling the laser device according to the stipulation of the control data for producing the incision figure in the eye.

11. Apparatus according to any one of Claims 1 to 5, wherein the incision figure further defines an auxiliary channel that extends from an incision face of the flap in the direction away from the flap, wherein the computer arrangement (28) is configured to produce, as a function of the ascertained position and orientation of at least those parts of the incision figure that define the flap, control data for producing the auxiliary channel in such a way that the auxiliary channel extends at least up into the region of the limbus of the eye, wherein the computer arrangement (28) is more particularly configured to produce the control data for producing the auxiliary channel in such a way that the auxiliary channel extends beyond the limbus of the eye.

12. Method according to any one of Claims 6 to 10, wherein the incision figure further defines an auxiliary channel that extends from an incision face of the flap in the direction away from the flap, wherein the method comprises: producing control data for producing the auxiliary channel as a function of the ascertained position and orientation of at least those parts of the incision figure that define the flap, in such a way that the auxiliary channel extends at least up into the region of the limbus of the eye, wherein the method more particularly comprises: producing the control data for producing the auxiliary channel in such a way that the auxiliary channel extends beyond the limbus of the eye.

## Revendications

1. Dispositif pour un système de traitement chirurgical oculaire assisté par un laser (10), comprenant:

   - une première unité de saisie d'image (36), qui est conçue pour saisir une première image (39) d'un œil à traiter (16),
   - un ensemble d'ordinateur (28), qui est conçu pour détecter par traitement d'image de la première image au moins une première caractéristique (40, 40') de l'œil (16), et pour déterminer une position et une orientation de la première caractéristique dans un système de coordonnées (S') du système de traitement (10), dans lequel l'ensemble d'ordinateur est en outre conçu pour déterminer une position et une orientation d'une figure de coupe (66') à produire dans l'œil (16) dans le système de coordonnées (S') du système de traitement (10) en fonction de la position et de l'orientation déterminées de la première caractéristique dans le système de coordonnés ainsi qu'en fonction d'une position et d'une orientation relatives déterminées au préalable d'au moins une deuxième caractéristique (64, 64') de l'œil (16) par rapport à la première caractéristique (40, 40'),

   **caractérisé en ce que**
   la figure de coupe (66') définit un volet cornéen avec une charnière de volet (68'), dans lequel l'ensemble d'ordinateur (28) est conçu pour déterminer la position et l'orientation d'un élément de figure de la figure de coupe définissant la charnière de volet (68') en fonction de la position et de l'orientation de la première caractéristique (40, 40') dans le système de coordonnées (S') du système de traitement (10) ainsi qu'en fonction de la position et de l'orientation relatives de la deuxième caractéristique (64, 64') par rapport à la première caractéristique (40, 40'), ladite au moins une deuxième caractéristique (64, 64') comprend une zone cornéenne à courbure astigmate et l'ensemble d'ordinateur (28) est conçu pour déterminer la position et l'orientation de la figure de coupe (66') en tenant compte d'une condition de position théorique prédéterminée entre le volet et la zone cornéenne à courbure astigmate, et/ou un axe de charnière ($Q_1'$-$Q_2'$) est associé à la charnière de volet (68') et un axe d'astigmatisme ($P_1'$-$P_2'$) est associé à la zone cornéenne à courbure astigmate, dans lequel l'ensemble d'ordinateur (28) est conçu pour déterminer la position et l'orientation de la figure de coupe (66') en tenant compte d'une condition de position théorique prédéterminée entre l'axe de charnière et l'axe d'astigmatisme, dans lequel en particulier la condition de position théorique entre l'axe de charnière ($Q_1'$-$Q_2'$) et l'axe d'astigmatisme ($P_1'$-$P_2'$) prédétermine une position des deux axes essentiellement perpendiculaire l'un à l'autre.

2. Dispositif selon l'une quelconque des revendications précédentes, dans lequel ladite au moins une première caractéristique (40, 40') est associée à un iris, à une pupille, à un limbe, à un agencement vasculaire scléral ou/et à une distribution d'épaisseur cornéenne de l'œil (16).

3. Dispositif selon l'une quelconque des revendications précédentes, comprenant un appareil de diagnostic (54) avec une deuxième unité de saisie d'image (56) pour la saisie d'une deuxième image (55) de l'œil à traiter (16), dans lequel l'appareil de diagnostic (54) est conçu pour détecter dans la deuxième image par traitement d'image ladite au moins une première caractéristique (40, 40') et pour produire des informations de caractéristique concernant une position et une orientation de chacune des deux caractéristiques, dans lequel en particulier:

   - l'ensemble d'ordinateur (28) est conçu pour déterminer sur la base des informations de caractéristique la position et l'orientation relatives de la deuxième caractéristique (64, 64') par rapport à la première caractéristique (40, 40'), et/ou
   - l'appareil de diagnostic (54) et la première unité de saisie d'image (36) sont associés à différentes stations de travail dans un cabinet médical, et/ou
   - une banque de données (62) est associée à l'appareil de diagnostic (54), pour y stocker les informations de caractéristique et/ou des informations déduites de celles-ci pour les associer à des informations d'identification des patients, et dans lequel l'ensemble d'ordinateur (28) a accès à la banque de données.

4. Dispositif selon l'une quelconque des revendications précédentes, dans lequel l'ensemble d'ordinateur (28) est conçu pour provoquer une représentation imagée de la figure de coupe (66'), qui illustre la position et l'orientation déterminées de la figure de coupe en relation avec la première caractéristique (40, 40') ou/et la deuxième caractéristique (64, 64') ou/et une zone cornéenne à ablater (70'), dans lequel en particulier:

   - l'ensemble d'ordinateur (28) est conçu pour présenter la représentation imagée sur un moniteur ou/et par superposition dans un trajet de faisceau d'observation d'un microscope opératoire, et/ou

- l'ensemble d'ordinateur (28) est conçu pour modifier la position ou/et l'orientation déterminée de la figure de coupe (66') selon une entrée d'utilisateur et pour modifier la représentation imagée de la figure de coupe selon la position ou/et l'orientation modifiée.

**5.** Dispositif selon l'une quelconque des revendications précédentes, dans lequel l'ensemble d'ordinateur (28) est conçu pour recevoir une confirmation introduite côté utilisateur pour la position et l'orientation de la figure de coupe (66') et pour produire en fonction de la réception de cette confirmation des données de commande pour un dispositif laser (12) et pour commander le dispositif laser selon ces données de commande pour la production de la figure de coupe dans l'œil.

**6.** Procédé de traitement chirurgical oculaire, comprenant les étapes suivantes:

- saisir une première image d'un œil à traiter,
- analyser la première image pour détecter au moins une première caractéristique de l'œil,
- déterminer une position et une orientation de la première caractéristique dans un système de coordonnées d'un système de traitement oculaire assisté par un laser,
- déterminer une position et une orientation d'une figure de coupe à produire dans l'œil dans le système de coordonnées du système de traitement en fonction de la position et de l'orientation déterminées de la première caractéristique dans le système de coordonnées ainsi qu'en fonction d'une position et d'une orientation relatives déterminées au préalable d'au moins une deuxième caractéristique de l'œil par rapport à la première caractéristique,

**caractérisé en ce que**
la figure de coupe définit un volet cornéen avec une charnière de volet et le procédé comprend l'étape suivante: déterminer la position et l'orientation d'un élément de figure de la figure de coupe définissant la charnière de volet en fonction de la position et de l'orientation déterminées de la première caractéristique dans le système de coordonnées du système de traitement ainsi qu'en fonction de la position et de l'orientation relatives de la première caractéristique par rapport à la deuxième caractéristique, ladite au moins une deuxième caractéristique comprend une zone cornéenne à courbure astigmate et le procédé comprend l'étape suivante: déterminer la position et l'orientation de la figure de coupe en tenant compte d'une condition de position théorique prédéterminée entre le volet et la zone cornéenne à courbure astigmate, et/ou

un axe de charnière est associé à la charnière de volet et un axe d'astigmatisme est associé à la zone cornéenne à courbure astigmate, dans lequel le procédé comprend l'étape suivante: déterminer la position et l'orientation de la figure de coupe en tenant compte d'une condition de position théorique prédéterminée entre l'axe de charnière et l'axe d'astigmatisme, dans lequel en particulier la condition de position théorique entre l'axe de charnière et l'axe d'astigmatisme prédétermine une position des deux axes essentiellement perpendiculaire l'un à l'autre.

**7.** Procédé selon la revendication 6, dans lequel ladite au moins une première caractéristique est associée à un iris, à une pupille, à un limbe, à un agencement vasculaire scléral ou/et à une distribution d'épaisseur cornéenne de l'œil.

**8.** Procédé selon la revendication 6, comprenant les étapes suivantes: saisir une deuxième image de l'œil à traiter, détecter ladite au moins une première caractéristique dans la deuxième image par traitement d'image, et déterminer des informations de caractéristique concernant une position et une orientation de chacune des deux caractéristiques, dans lequel le procédé comprend en outre les étapes suivantes:

- déterminer la position et l'orientation relatives de la deuxième caractéristique par rapport à la première caractéristique sur la base des informations de caractéristique, et/ou
- préparer des systèmes de caméras séparés pour la saisie de la première image et de la deuxième image, dans lequel les systèmes de caméras sont associés en particulier à différentes stations de travail dans un cabinet médical, et/ou
- mémoriser les informations de caractéristique ou/et des informations déduites de celles-ci dans une banque de données en association avec des informations de patients.

**9.** Procédé selon la revendication 6, comprenant les étapes suivantes: produire une représentation imagée de la figure de coupe, qui illustre la position et l'orientation déterminées de la figure de coupe en relation avec la première caractéristique ou/et la deuxième caractéristique ou/et une zone cornéenne à ablater, dans lequel le procédé comprend en outre les étapes suivantes:

- présenter la représentation imagée sur un moniteur, et/ou
- superposer la représentation imagée dans un trajet de faisceau d'observation d'un microscope opératoire, et/ou
- modifier la position ou/et l'orientation déterminées de la figure de coupe selon une entrée de l'utilisateur et modifier la représentation imagée de la figure de coupe selon la position ou/et l'orientation modifiée de celle-ci.

10. Procédé selon la revendication 6, comprenant les étapes suivantes : recevoir une confirmation introduite côté utilisateur pour la position et l'orientation de la figure de coupe, produire des données de commande pour un dispositif laser en fonction de la réception de cette confirmation, et commander le dispositif laser selon les données de commande pour la production de la figure de coupe dans l'œil.

11. Dispositif selon l'une quelconque des revendications 1 à 5, dans lequel la figure de coupe définit en outre un canal auxiliaire, qui s'étend depuis une face de coupe du volet en s'éloignant du volet, dans lequel l'ensemble d'ordinateur (28) est conçu pour produire, en fonction de la position et de l'orientation déterminées au moins des parties de la figure de coupe, qui définissent le volet, des données de commande pour la production du canal auxiliaire, de telle manière que le canal auxiliaire s'étende au moins jusque dans la région du limbe de l'œil, dans lequel l'ensemble d'ordinateur (28) est conçu en particulier pour produire les données de commande pour la production du canal auxiliaire, de telle manière que le canal auxiliaire s'étende jusqu'au-delà du limbe de l'œil.

12. Procédé selon l'une quelconque des revendications 6 à 10, dans lequel la figure de coupe définit en outre un canal auxiliaire, qui s'étend depuis une face de coupe du volet en s'éloignant du volet, dans lequel le procédé comprend l'étape suivante : produire des données de commande pour la production du canal auxiliaire en fonction de la position et de l'orientation déterminées au moins des parties de la figure de coupe, qui définissent le volet, de telle manière que le canal auxiliaire s'étende jusque dans la région du limbe de l'œil, dans lequel le procédé comprend en particulier l'étape suivante : produire les données de commande pour la production du canal auxiliaire, de telle manière que le canal auxiliaire s'étende jusqu'au-delà du limbe de l'œil.

# FIG 1

EP 2 729 099 B1

FIG 2a

FIG 2b

# FIG 3

```
                    ┌─────────────────────┐
                    │ Irisaufnahme mit    │── S104
                    │ einem Diagnosegerät │
                    └─────────────────────┘                          102
                             │                    S110                 ↙
              ┌──────────────┴────────────┐         │
     S106 ~  / Bilddaten mit              /      / Diagnosedaten /
            / individuellen Merkmalen    /       └──────────────┘
           └────────────────────────────┘                    │
                             │                                │    z. Bsp.
                    ┌────────┴────────┐── S108                │    Karatometerwerte
                    ┆ Merkmalsextraktion ┆                    │
                    ┆ (optional)        ┆                     │
                    └─────────────────┘                       │
                             │                                │
              ┌──────────┐── S112          ┌──────────┐── S112
              │ Datenträger │               │ Datenbank │
              └──────────┘                  └──────────┘
```

S100

```
┌──────────────────────────────────────────────────────────────────────┐
│ Femtosekundenlaser                                                     │
│                                                                        │
│   ┌──────────────┐              ┌──────────────┐                       │
│   │ Auge andocken │─── S116 ──→ │ Irisaufnahme │←─ ─ ─ ─               │
│   └──────────────┘              └──────────────┘                       │
│          S114                          │                               │
│                                 ┌──────────────┐     Automatisierte    │
│                          S118 ~ │ Merkmalsextraktion │  Angleichung der │
│                                 │ (optional)    │     Diagnosedaten     │
│                                 └──────────────┘     zur aktuellen      │
│                                        │             Augenposition      │
│                                 ┌──────────────┐                        │
│                         S120 ~  │ [icons]      │                        │
│                                 └──────────────┘                        │
│                                        │           / Parameter         /│
│    S128                                │          / Ablationsprofil    /│
│   ┌──────────────┐       ┌──────────────┐         └─────────────┘      │
│   │ Parameter in GUI │←──│ Berechnung der │── S124      S122            │
│   │ einstellen    │      │ idealen Hinge- │                            │
│   └──────────────┘       │ und Flapposition │                          │
│     S126                 └──────────────┘                              │
│        │                        │                                      │
│   ( Einzeichnen der )      ( Einzeichnen der )── S126                  │
│   ( Flapkontur in GUI )    ( Flapkontur in HUD )                       │
│        │                        │           Ja   S132      S130        │
│   ┌──────────────┐   Nein    ◇─────────◇         ┌──────────────┐      │
│   │ Behandlungs-  │←─────────◇Modifikation?◇─ ─ ─│ Modifikation der │   │
│   │ durchführung  │          ◇─────────◇         │ Flapposition  │     │
│   └──────────────┘     S134                      └──────────────┘      │
│        S136                                                            │
└──────────────────────────────────────────────────────────────────────┘
```

FIG 4

FIG 5

# FIG 6

EP 2 729 099 B1

FIG 7

EP 2 729 099 B1

FIG 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2003223037 A1
- EP 1767174 A2
- WO 2011035063 A1
- US 6866661 B2